(19) Europäisches Patentamt
European Patent Office
Office européen des brevets

(11) **EP 3 447 153 B1**

(12) **EUROPEAN PATENT SPECIFICATION**

(45) Date of publication and mention
of the grant of the patent:
**27.10.2021 Bulletin 2021/43**

(51) Int Cl.:
***C12Q 1/6806*** (2018.01)

(21) Application number: **17203198.1**

(22) Date of filing: **23.11.2017**

(54) **METHOD FOR DETERMINING THE QUALIFICATION OF A SAMPLE FOR FETAL ANEUPLOIDY STATUS DETERMINATION IN NON-INVASIVE PRENATAL TESTING**

VERFAHREN ZUR BESTIMMUNG DER EIGNUNG EINER PROBE ZUR BESTIMMUNG DES STATUS VON FÖTALER ANEUPLOIDIE IN NICHTINVASIVEN PRÄNATALEN TESTS

PROCÉDÉ POUR DÉTERMINER LA QUALIFICATION D'UN ÉCHANTILLON DE DÉTERMINATION DE L'ÉTAT D'ANEUPLOÏDIE F TALE LORS D'ESSAIS PRÉNATAUX NON INVASIFS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.08.2017 SK 862017**

(43) Date of publication of application:
**27.02.2019 Bulletin 2019/09**

(73) Proprietor: **Comenius University in Bratislava 814 99 Bratislava 1 (SK)**

(72) Inventors:
• **Grendár, Marian**
**97404 Banská Bystrica (SK)**
• **Loderer, Dusan**
**97662 Brusno (SK)**
• **Lasabová, Zora**
**03608 Martin (SK)**
• **Danko, Ján**
**03601 Martin (SK)**

(74) Representative: **Schiweck Weinzierl Koch Patentanwälte Partnerschaft mbB Ganghoferstraße 68 B 80339 München (DE)**

(56) References cited:
**WO-A1-2016/057901**

• **DAPHNE M. VAN BEEK ET AL: "Comparing methods for fetal fraction determination and quality control of NIPT samples : Comparing fetal fraction and quality control tools for NIPT", PRENATAL DIAGNOSIS, vol. 37, no. 8, 10 July 2017 (2017-07-10), pages 769-773, XP055472942, GB ISSN: 0197-3851, DOI: 10.1002/pd.5079**
• **FRANCESCO FIORENTINO ET AL: "The importance of determining the limit of detection of non-invasive prenatal testing methods : The importance of determining the limit of detection of NIPT methods", PRENATAL DIAGNOSIS, vol. 36, no. 4, 21 February 2016 (2016-02-21), pages 304-311, XP055473015, GB ISSN: 0197-3851, DOI: 10.1002/pd.4780**
• **C. ZHAO ET AL: "Detection of Fetal Subchromosomal Abnormalities by Sequencing Circulating Cell-Free DNA from Maternal Plasma", CLINICAL CHEMISTRY, vol. 61, no. 4, 20 February 2015 (2015-02-20), pages 608-616, XP055215005, ISSN: 0009-9147, DOI: 10.1373/clinchem.2014.233312**
• **MARKUS STUMM ET AL: "Diagnostic accuracy of random massively parallel sequencing for non-invasive prenatal detection of common autosomal aneuploidies: a collaborative study in Europe", PRENATAL DIAGNOSIS, vol. 34, no. 2, 12 December 2013 (2013-12-12), pages 185-191, XP055344715, GB ISSN: 0197-3851, DOI: 10.1002/pd.4278**

EP 3 447 153 B1

- **L. F. JOHANSSON ET AL: "Novel Algorithms for Improved Sensitivity in Non-Invasive Prenatal Testing", SCIENTIFIC REPORTS, vol. 7, no. 1, 12 May 2017 (2017-05-12), XP055472801, DOI: 10.1038/s41598-017-02031-5**
- **HAN ZHANG ET AL: "Statistical Approach to Decreasing the Error Rate of Noninvasive Prenatal Aneuploid Detection caused by Maternal Copy Number Variation", SCIENTIFIC REPORTS, vol. 5, no. 1, 4 November 2015 (2015-11-04), pages 1-9, XP055407852, DOI: 10.1038/srep16106**
- **TANJA SCHLAIKJAER HARTWIG ET AL: "Discordant non-invasive prenatal testing (NIPT) - a systematic review : Discordant NIPT cases - a review", PRENATAL DIAGNOSIS, vol. 37, no. 6, 1 June 2017 (2017-06-01), pages 527-539, XP055472773, GB ISSN: 0197-3851, DOI: 10.1002/pd.5049**
- **C. H. YEANG ET AL: "Genome-wide normalized score: a novel algorithm to detect fetal trisomy 21 during non-invasive prenatal testing", ULTRASOUND IN OBSTETRICS AND GYNECOLOGY, vol. 44, no. 1, 12 June 2014 (2014-06-12), pages 25-30, XP055407854, GB ISSN: 0960-7692, DOI: 10.1002/uog.13377**

**Description**

FIELD OF THE INVENTION

This invention relates to Non-Invasive Prenatal Testing (NIPT). Particularly, the present invention relates to methods for determining whether or not a sample obtained from a pregnant subject is qualified for determining the fetal aneuploidy status. The present invention further relates to methods for determining the limit of detection (LoD) $f_{LoD}^{T}$ for trisomy and the limit of detection (LoD) $f_{LoD}^{M}$ for monosomy, respectively, on a chromosome within the read-counting approach to the non-invasive prenatal testing.

BACKGROUND OF THE INVENTION

The chromosomal read counting approach to Noninvasive Prenatal Testing (NIPT) utilizes in most cases Whole Genome Sequencing (WGS) of the cell-free DNA (cfDNA) (Lo et al., The Lancet 350, 485-487 (1997)) and a subsequent bioinformatic pipeline (Bayindir et al., Eur. J. Hum. Genet. 23, 1286-1293 (2015); Porreco et al., Am. J. Obstet. Gynecol. 211, 365-e1 (2014); Thung et al., Expert. Rev. Mol. Diagn. 15, 111-124 (2015); US2016224724; EP2981921) that leads the total number of aligned, preprocessed, normalized reads in a chromosome or a subset of the chromosome. The number of reads, also referred to herein as the "read representation of a chromosome" or "chromosome representation", is then in most cases usually compared to a threshold, derived from euploid pregnancies. This way it is usually decided whether the sample under investigation is aneuploid or euploid. There are several variations of the just-described principle (Tamminga et al., Adv. Clin. Chem. 74, 63-102 (2016)), involving different instances of the alignment procedure (Bayindir, loc cit; Chiu et al., Proc. Natl. Acad. Sci. 105, 20458-20463 (2008)), preprocessing (Jensen et al., PloS One 8, e57381 (2013); Chandrananda et al., PloS One 9, e86993 (2014)) and normalization (Sehnert et al., Clin. Chem. 57, 1042-1049 (2011); Zhao et al., Clin. Chem. 61, 608-616 (2015); Yeang et al., Ultrasound Obstet. & Gynecol. 44, 25-30 (2014)), as well as of the subsequent decision making (Bayindir, loc cit; Tynan, loc cit; Johansson et al., Sci. Reports 7 (2017); Sikkema-Raddatz et al., Sci. Reports 6, 38359 (2016); Balslev-Harder et al., Prenat. Diagn. DOI 10.1002/pd.5116).

The "no call (or failure) rate" is an important characteristics of any diagnostic, testing, or screening method. Failures of NIPT are primarily caused by low fetal fraction, which is the amount of the cell-free DNA (cfDNA) in the maternal blood that is of the fetal origin. If fetal fraction is below a value, known as the Limit of Detection (LoD), then in methods known in the arts NIPT cannot determine the aneuploidy status. In the art, the LoD of conventional NIPT for the common aneuploidies is found to be around 4% (Palomaki et al., Genet. Medicine 13, 913-920 (2011); Canick et al., Prenat. Diagn. 33, 667-674 (2013); Ehrich et al., Am. J. Obstet. Gynecol. 204, 205-e1 (2011); Norton et al., Am. J. Obstet. Gynecol. 207, 137—e1 (2012); Ashoor et al, Ultrasound Obstet. & Gynecol. 41, 26-32 (2013)). The LoD can also be determined experimentally (Fiorentino et al., Prenat. Diagn. 36, 304-311 (2016); URL http://dx.doi.org/10.1002/pd.4780). In Van Beek et al., PRENATAL DIAGNOSIS 8, 769-773 (2017) available analysis methods for determining fetal fraction on single read next generation sequencing data are compared.

The "no call rate" of NIPT is rather variable (Mackie et al., BJOG: An Int. J. Obstet. & Gynaecol. 124, 32-46 (2017); Gil et al., Fetal Diagn. Ther. 35, 156-173 (2014)). The no call rate negatively affects sensitivity and specificity of NIPT (Taylor-Phillips et al., BMJ Open 6, e010002 (2016)). In methods known in the art, if the fetal fraction is below the LoD, then the analyzed sample (fetus, pregnancy) is reported as 'no call', regardless of the read representation of a chromosome (Tamminga et al., Advances in clinical chemistry.;74:63-102 (2016)). Such "no call" sample is then usually disregarded and discarded. Thus, repeated sampling is necessary, which is undesirable both for the subject where the sample is taken from, and for the tester.

Accordingly, the technical problem underlying the present invention was to comply with the objectives set out above. The technical problem has been solved by means and methods as described herein, illustrated in the examples and as defined in the claims.

DESCRIPTION OF THE INVENTION

In one aspect of the present invention, it was an objective to find ways of increasing the detection rate of NIPT or to find ways to determine the suitability of a given sample (e.g., pregnant/maternal; fetal subject) for determining the fetal aneuploidy (particularly trisomy, monosomy) status, using both fetal fraction and read representation of a chromosome.

Accordingly, in one aspect the present invention relates to a NIPT method for determining whether or not a sample obtained from a pregnant subject, preferably a human subject is qualified for determining the fetal trisomy status of a chromosome, comprising determining whether

(a)

$$f \leq f_{LoD}^{T} \qquad \text{(1a)}$$

(b)

$$t \geq (1 + 0.5 * f) * t_{min}^{E} \qquad \text{(1b)}$$

(c)

$$t \leq t_{max}^{E} \qquad \text{(1c)}$$

wherein $f$ is the fetal fraction,
t is the read representation of a chromosome,
$f_{LoD}^{T}$ is the limit of detection (LoD) for trisomy of the chromosome,
$t_{min}^{E}$ is the minimal value of the read representation of the chromosome in a set of euploid samples, and
$t_{max}^{E}$ is the maximal value of the read representation of the chromosome in a set of euploid samples;
wherein, if all of (a) to (c) are fulfilled, said sample is not qualified for determining the fetal trisomy status of the chromosome, or
wherein, if any one of (a) to (c) is not fulfilled, said sample is qualified for determining the fetal trisomy status of the chromosome.

In another aspect, the present invention relates to a NIPT method for determining whether or not a sample obtained from a pregnant subject, preferably a human subject is qualified for determining the fetal monosomy status of a chromosome, comprising determining whether

(a)

$$f \leq f_{LoD}^{M} \qquad \text{(3a)}$$

(b)

$$t \leq (1 - 0.5 * f) * t_{max}^{E} \qquad \text{(3b)}$$

(c)

$$t \geq t_{min}^{E} \qquad \text{(3c)}$$

wherein $f$ is the fetal fraction,
t is the read representation of the chromosome,
$f_{LoD}^{M}$ is the limit of detection (LoD) for a monosomy of the chromosome,
$t_{min}^{E}$ is the minimal value of the read representation of the chromosome in a set of euploid samples, and
$t_{max}^{E}$ is the maximal value of the read representation of the chromosome in a set of euploid samples;
wherein, if all of (a) to (c) are fulfilled, said sample is not qualified for determining the fetal monosomy status of the chromosome, or
wherein, if any one of (a) to (c) is not fulfilled, said sample is qualified for determining the fetal monosomy status of the chromosome.

In one embodiment of the present invention, the pregnant subject is mammal, preferably a human subject.

The present invention generally provides a NIPT method for determining the qualification of a sample for the fetal aneuploidy status determination in the read-counting approach to non-invasive prenatal testing, whereas the invention takes into account both fetal fraction and read representation of a chromosome.

In context with the present invention, the (maternal) sample may be any sample of a pregnant subject (e.g., mammal, preferably human) comprising nucleic acid molecules (e.g., DNA or RNA, preferably DNA) or fragments or segments thereof, of fetal origin. Examples may comprise *inter alia* blood, particularly plasma or serum (preferably plasma). The preparation of serum or plasma from the maternal blood sample may be carried out by standard techniques known in the art. The serum or plasma may then be subjected to a nucleic acid extraction process. Possible methods include *inter alia* the controlled heating method described by Frickhofen and Young (1991) as known in the art. Another suitable serum and plasma extraction method is proteinase K treatment followed by phenol/chloroform extraction. Serum and plasma nucleic acid extraction methods allowing the purification of DNA or RNA from larger volumes of maternal sample increase the amount of fetal nucleic acid material for analysis and thus improve the accuracy. A sequence-based enrichment method could also be used on the maternal serum or plasma to specifically enrich for fetal nucleic acid sequences.

As also described above, NIPT (non-invasive prenatal testing) is a non-invasive screening and testing method which allows *inter alia* determining the fetal aneuploidy status for selected chromosomes (e.g., 21, 18, 13, 8, 1, 5, X); cf. also EP 994963A or EP 1981995A. NIPT is based on sequencing (Norwitz et al., Rev Obstet Gynecol (2013), 6(2): 48-62) cell-free DNA (cfDNA) in suitable samples, e.g., maternal plasma samples. Common approaches for NIPT are also described in, e.g., EP 2183693A and EP 2514842A. Such cfDNA is circulating in maternal blood and can be measured by several sequencing methods, particularly MPS (massive parallel sequencing) methods as known in the art and also described herein. The cfDNA also comprises nucleic acid (particularly DNA) molecules from the fetus of a pregnant subject (preferably human), also referred to as cell-free fetal DNA (cffDNA) (Barrett et al., PloS One (2011), 6: e25202) and cell-free maternal DNA (cfmDNA), respectively. The amount of cffDNA in relation to the total amount of the maternal cfDNA in the same sample is also referred to as fetal fraction ($f$), which is determined in accordance with the present invention as further described and exemplified herein. Such cffDNA may be present as segments or fragments and sequenced by MPS methods and the resulting reads may be aligned to a human genome and counted, e.g., by methods known in the art and described and exemplified herein. This way, the read representation of a chromosome ($t$), i.e. number of reads which are mapped to a particular chromosome or subregions thereof and subjected to preprocessing and normalization, may be determined in accordance with the present invention. The purpose in context with the present invention is to determine whether the read representation of a chromosome (e.g., 21, 18, 13, 1, 5, or X) is higher or lower compared to a normal (i.e. euploid or disomic) reference, which would then be indicative for an aneuploidy (e.g., trisomy or monosomy, respectively) of the fetal chromosome set (Norwitz et al., Rev Obstet Gynecol (2013), 6(2): 48-62). Thus, it is possible to determine the aneuploidy status of a fetus with respect to that selected chromosome(s), e.g. to determine a trisomy or monosomy for such chromosome(s). For example, if the number of a chromosome-specific reads exceeds the threshold that represents a normal (euploid or disomic) chromosome status, the result is reported as positive for trisomy for that chromosome (Norwitz et al., Rev Obstet Gynecol (2013), 6(2): 48-62). The same holds true vice versa for falling below such threshold which is then reported as positive for monosomy for that chromosome.

It is also possible in accordance with the present invention to amplify fetal DNA molecules from the sample (e.g., blood, preferably serum or plasma, preferably plasma). Standard nucleic acid amplification systems can be used, including the polymerase chain reaction (PCR), the ligase chain reaction, nucleic acid sequence based amplification (NASBA), branched DNA methods, and so on. Preferred amplification methods involve PCR. Typically, the amplification uses at least one foetal sequence specific oligonucleotide primer. Generally, the nucleic acid may be detected by means of a sequence specific probe.

The LoD may be determined by methods known on the art and also described herein (e.g., Fiorentino et al., Prenat. Diagn. 36, 304-311 (2016). URL http://dx.doi.org/10.1002/pd.4780). The LoD may for example be determined experimentally as known in the art and as described herein. The LoD may be different for each respective chromosome for which an aneuploidy (e.g., monosomy or trisomy) shall be determined in accordance with the methods of the present invention as described and provided herein. In conventional NIPT methods known in the art, the set of "no call" samples comprises all samples that have fetal fraction f below the LoD. Such "no call" samples are then usually disregarded and discarded. That is, in such conventional NIPT methods a sample with fetal fraction below LoD is considered as "no call" and thus disregarded and discarded, regardless of its read representation of a chromosome t.

The present invention provides NIPT methods which allow a more precise and reliable determination on whether a given sample is qualified for determining the fetal aneuploidy (e.g., tri- or monosomy) status by taking into account both fetal fraction f and read representation of a chromosome t and, thus, shrinking the set of "no call" samples which allows avoiding unreasonable discharge of qualified samples.

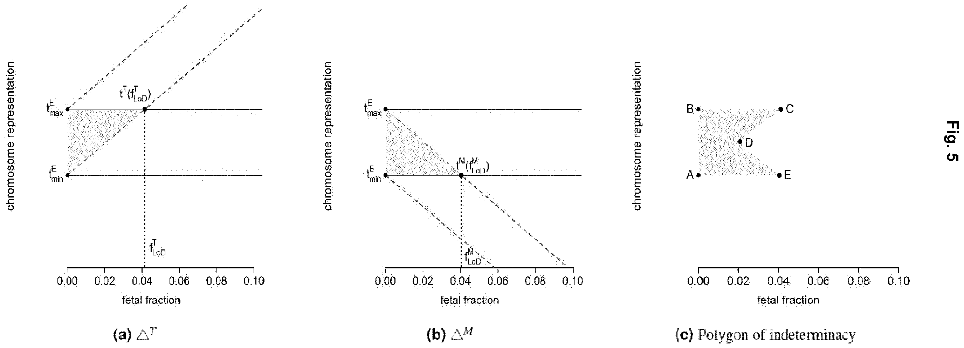

In context with the present invention, it has been surprisingly found that both fetal fraction f and read representation of a chromosome t must be taken into account to reliably determine the qualification of a given sample to determine the fetal aneuploidy (e.g., tri- or monosomy) status of the chromosome. As shown herein, the set of values (f,t) for which the read-counting based NIPT methods cannot call the fetal aneuploidy status of a sample forms a union of two triangles, which resembles the stylized letter ,K' (see Figure 5). The "no call" set according to the method described and provided in context with the present invention is much smaller than the "no call" set of the conventional NIPT. The method of the present invention, coupled with fetal fraction estimation by methods known in the art and also described and exemplified herein (e.g., seqFF; cf. Kim et al., Prenat. Diagn. (2015), 35: 810-815), attained a substantive reduction of "no cell rate", e.g., for the common trisomies the "no call rate" could be 0%-1% using the method of the present invention, in comparison to 5%-8% of the conventional NIPT (see Table 2).

The inventive method for the fetal aneuploidy (e.g., tri- or monosomy) status determination in NIPT utilizes both fetal fraction $f$ and read representation of a chromosome $t$. According to the present invention, a fetal aneuploidy status cannot be reliably determined if fetal fraction f and read representation of a chromosome t belong to the Triangle of Uncertainty for Trisomy (TUT) for the chromosome or to the Triangle of Uncertainty for Monosomy (TUM) for the chromosome. The present method for the fetal aneuploidy determination increases the detection rate of NIPT, while retaining its high sensitivity and specificity.

"Circulating cell free DNA (cfDNA)" as used herein comprises (fragments of) nucleic acid (DNA, RNA; preferably DNA) molecules of different length, which are not organized in cell nuclei and not separated by membrane from outer medium, but which are localized in liquid medium within a given subject (preferably mammal, more preferably human) such as urine, plasma, or serum (preferably plasma). cfDNA can be found in samples from a pregnant subject (preferably mammal, preferably human), e.g., from maternal plasma samples. The skilled person is aware of methods to measure and count nucleic acid (particularly DNA) molecules in (plasma) samples, particularly reads from cfDNAas described herein. For example, MPS (massive parallel sequencing) methods as known in the art and also described and exemplified herein allow fast counting of myriads of nucleic acid (particularly DNA) molecules, e.g. reads as described herein (Lo, Open Biol. (2012), 2(6): doi 10.1098/rsob.120086). Further methods for detecting cfDNA, cffDNA or cfmDNA are known in the art and described, e.g., in Nigam et al., JIMSA (2012), 25: 119-200.

"Circulating cell free fetal DNA (cffDNA)" as used herein comprises cfDNA fragments of fetal origin in a given sample of the pregnant mother of the fetus (preferably mammal, more preferably human), preferably cfDNA fragments of fetal origin in the plasma of a pregnant human. The skilled person is aware of methods to measure and count nucleic acid (particularly DNA) molecules in (plasma) samples, particularly reads from cffDNA as described herein. For example, MPS (massive parallel sequencing) methods as known in the art and also described and exemplified herein allow fast counting of myriads of nucleic acid (particularly DNA) molecules, e.g. reads as described herein (Lo, Open Biol. (2012), 2(6): doi 10.1098/rsob.120086).

"Circulating cell free maternal DNA (cfmDNA)" or "maternal cfDNA" as used herein comprises cfDNA fragments of maternal origin in a given sample of the pregnant mother, preferably cfDNA fragments of maternal origin in the plasma of a pregnant human. The skilled person is aware of methods to measure and count nucleic acid (particularly DNA) molecules in (plasma) samples, particularly reads of cfDNA, cfmDNA and cffDNA as described herein. For example, MPS (massive parallel sequencing) methods as known in the art and also described and exemplified herein allow fast counting of millions and billions nucleic acid (particularly DNA) molecules, e.g. reads as described herein (Lo, Open Biol. (2012), 2(6): doi 10.1098/rsob.120086).

"Fetal fraction ($f$)" as used herein means the amount of cffDNA in relation to the total amount of the maternal cfDNA in the same sample (expressed as percentage). The fetal fraction (also known as fractional fetal DNA concentration) can be measured by means and methods known in the art. The fetal fraction may be measured, e.g., in maternal blood plasma samples from pregnant women, where the specific signal originating from Y chromosome may be used to deduce the fetal DNA fractions in pregnancies carrying male fetuses (Chiu at al., BMJ (2011), 342: c7401; Hudecova et al., PLoS One (2014), 9: e88484; also known as "ffY method"). However, this may not be suitable for pregnancies carrying female fetuses, where alternative approaches such as the targeted sequencing, e.g., SNP (single nucleotide polymorphism) may be used to calculate the ratio of the fetal specific alleles to the shared alleles to infer the fetal DNA fraction. Yet, this approach requires prior knowledge of the genotype of the fetus (Liao et al., Clin Chem (2011), 57: 91-101). Further methods different from the SNP approach are known in the art and described, *inter alia,* in Lo et al., Sci Transl Med (2010), 2: 61ra91; Yu et al., PNAS (2014), 111: 8583-8588. Other methods for determining fetal fractions of DNA in maternal blood plasma samples (independent of the determining maternal or fetal genotypes) are described *inter alia* in WO 2017/050244. Further methods are known in the art and also described and exemplified herein and include "seqFF" (Kim et al., Prenat. Diagn. (2015), 35: 810-815) and "seqFFY" as described herein in the Examples (see Example 4). In one embodiment of the present invention, the fetal fraction ($f$) is measured using seqFF or seqFFY.

"Massive Parallel Sequencing (MPS)" as used herein means simultaneous, massive, parallel sequencing of an extremely large amount of DNA molecules. It is also referred to in the art and herein as "next generation sequencing" or "NGS", or as "second generation sequencing" (for some sequencing methods also "third generation sequencing"). As readily clear to the skilled person, the sequencing data (i.e., reads) may be mapped to the reference (e.g., human) genome using bioinformatic tools as known in the art and also described and exemplified herein (e.g., Bowtie2 as described in Langmead et al., Nat Methods (2012), 9: 357-359). Unmapped, duplicate, low quality reads may be filtered out by suitable tools as known in the art and exemplified herein in Example 4. The reads mapped (i.e., aligned) to a particular chromosome (e.g., 21, 18, 13, 5, 1, X) may be counted and the reads count may then be compared to the number of reads in the chromosome of one or more samples which are euploid in the chromosome (Norwitz et al., Rev Obstet Gynecol (2013), 6(2): 48-62). Examples for MPS methods comprise *inter alia* Polony sequencing, 454 (pyro)sequencing, Ilumina dye sequencing, SOLiD sequencing, DNA nanoball sequencing, SMRT sequencing, Nanopore sequencing, and others (cf., e.g., WO 9844151; Duncan et al., Diagn Mol Pathol (2017), doi. org/10.1016/B978-0-12-800886-7.00003-0; Voelkerding et al., Clin Chem (2009), 55(4): 641-658; Wheeler et al., Nature (2008), 452(7189): 872-876; Canard et al., Gene (1994), 148(1): 1-6; Feng et al., Mol Ecol Res (2015), 16(1): 91-102; Schuster, Nat Methods (2008), 51(1): 16-18; WO 06/084132; DRManac et al., Science (2010), 327(5961): 78-81; Porreca, Nat Biotechnol (2010), 28(1): 43-44; Harris et al., Science (2008), 320(5872): 106-109; Levene et al., Science (2003), 299(5607): 982-686; Eid et al., Science (2009), 323(5910): 133-138; Niedringhaus et al., Anal Chem (2011), 83(12): 4327-4341; Greininger et al., Genome Medicine (2015) 7: 99).

"Read" as used herein means a sequence of segment or fragment of DNA, for example which may be derived from a particular chromosome (e.g., 21, 18, 13, 5, 1, or X), obtained by sequencing, e.g. by MPS methods; cf. Bayindir et al., Eur. J. Hum. Genet. 23, 1286-1293 (2015); Porreco et al., Am. J. Obstet. Gynecol. 211, 365-e1 (2014); Thung et al., Expert. Rev. Mol. Diagn. 15, 111-124 (2015); US2016224724; EP2981921. In accordance with the method described and provided herein, usually such reads of cfDNA, cfmDNA and cffDNA are obtained by MPS methods as known in the art and described herein.

"Read representation of a chromosome (*t*)" or "chromosome representation *(t)*" as used herein means the number of reads mapped to a particular chromosome or subregions thereof (also referred to herein as "(chromosomal) read counts") and optionally filtered, preprocessed and normalized. Normalization can be done as described herein and further shown, e.g., in Bayindir et al., Eur. J. Hum. Genet. 23, 1286-1293 (2015); Porreco et al., Am. J. Obstet. Gynecol. 211, 365-e1 (2014); Thung et al., Expert. Rev. Mol. Diagn. 15, 111-124 (2015); US2016224724; EP2981921; McCullugh et al., PloS One (2014): MaterniT21). Normalization methods are known in the art and also described and exemplified herein; cf. Example 4. Suitable methods may comprise *inter alia* those described in the examples herein and those described in, e.g., Jensen et al., PloS One (2013), 8: e57381 and Kim et al., Prenat. Diagn. (2015), 35: 810-815.

"Limit of Detection (LoD)" as used herein means the value of fetal fraction below which the fetal aneuploidy status is considered not to be determinable (callable) in a conventional NIPT. The LoD can be determined for instance experimentally as known in the art and also described herein (see, e.g., Fiorentino et al., Prenat. Diagn. 36, 304-311 (2016). URL http://dx.doi.org/10.1002/pd.4780). A commonly used value of LoD is about 4%. According to the present invention, each chromosome (e.g., 13, 18, 21, X, 1, 5) has its own $f_{LoD}^{M}$ or $f_{LoD}^{T}$ for detection of monosomy or trisomy, respectively. For example, the LoD for trisomy of chromosome 21 may be about 3 to 4.5%. As another example, the LoD for trisomy of chromosome 18 may be about 3.5 to 6.5%. As another example, the LoD for trisomy of chromosome 13 may be about 3.2 to 5.8%. As another example, the LoD for monosomy of chromosome 21 or 18 may be about 3 to 4%. As another example, the LoD for monosomy of chromosome 13 may be about 3.5 to 4.5%. Also, for detection of trisomy, $f_{LoD}^{T}$ can be decreased by decreasing the range of euploid read representation of a chromosomes ( $t_{max}^{E}$ - $t_{min}^{E}$ ) and/or by increasing $t_{min}^{E}$. The same applies *mutatis mutandis* for monosomy and $f_{LoD}^{M}$, respectively. The LoD for determining particular chromosome aneuploidies may also be dependent on the method for normalization of the reads as shown and described herein; cf. Example 4.

"No call' sample" as used herein means a sample for which the fetal aneuploidy status cannot be determined.

"Conventional NIPT" as used herein particularly comprises a method for the fetal aneuploidy status determination where a sample is considered as 'no call' if its fetal fraction is below LoD, regardless of the read representation of a chromosome.

As used herein, the term "aneuploidy" means the presence of an abnormal number of (portions of) chromosomes in a cell and comprises any kind of deviation from the diploid chromosome set where two homologous copies of each autosome (e.g., for human beings, chromosomes 1 to 22) and two allosomes (sex chromosomes; e.g., for mammals such as humans two copies of the X chromosome for females and one copy of the X and one copy of the Y chromosome for males) are present in a cell of a given subject (e.g., fetus; human fetus), where one copy of each chromosome is received from the mother and one from the father, respectively (for sex chromosomes in mammals like humans, one X is received from the mother, while the other X or the Y, respectively, is received from the father of the individuum). The term "aneuploidy" may also be used herein interchangeably with the terms "numerical chromosomal abnormality", "numerical chromosomal aberration" or "numerical chromosomal disorder" as known in the art. The term "aneuploidy" as used herein also comprises abnormal number of individual chromosomes, e.g. only one copy of a specific chromosome (i.e. a monosomy; e.g., monosomy of chromosome 13, 18, 21 or X), or three copies of a specific chromosome (i.e. a trisomy; e.g., trisomy of chromosome 13, 18, 21 or X). Accordingly, as used herein, the term "aneuploidy" also comprises "trisomy", tetrasomy" or "monosomy" (preferably tri- or monosomy). Analoguously, the term "euploidy" describes the normal state of a chromosome set, e.g. for mammals like humans, two copies of each autosome and two sex chromosomes (XX or XY) (i.e. for mammals diploidy of set of n=23 chromosomes (22 autosomes and 1 sex chromosome), or the status of all chromosomes is disomic (for males normally X and Y are present as sex chromosomes as readily understood by the skilled person). Likewise, the presence of two copies of a given chromosome is referred to heren and also in the art as "disomy" (e.g., two copies of chromosomes 13, 18, 21 or X (for X, this means XX = female).

"Trisomy" as used herein means that for at least one chromosome, there are three copies of a chromosome present in the set of chromosomes of a given subject (e.g., fetal set of chromosomes). For example, in context with the present invention, trisomy may comprise a trisomy for one or more of chromosomes 21 ("trisomy 21"), 18, 13, 9, 8, 22 or X, where three copies of at least one (preferably only one) of said chromosomes are present in a cell or all cells of the subject (fetus, e.g., human fetus), or a XXY or XYY trisomy where three sex chromosomes are present. In one embodiment of the present invention, "trisomy" means three copies of chromosome 21 ("Down syndrome", "trisomy 21"), 18 ("Edwards syndrome" , "trisomy 18"), 13 ("Patau syndrome", "trisomy 13"), 8 ("Warkany syndrome 2" , "trisomy 8"), X ("Triple X syndrome" , "trisomy X"), or three sex chromosomes other than XXX, namely XYY ("Jacobs syndrome") or XXY ("Klinefelter syndrome"), preferably trisomy 21, 18, 13 or X, or three sex chromosomes other than XXX, namely XYY ("Jacobs syndrome") or XXY ("Klinefelter syndrome"). In a particular embodiment of the present invention, the fetal trisomy status is trisomy 13, 18, 21 or X.

In one embodiment of the present invention, relating to the NIPT method for determining whether or not a sample obtained from a pregnant subject, preferably a human subject is qualified for determining the fetal trisomy status, the fetal trisomy status is trisomy 21, 13, 18 or X.

"Monosomy" as used herein means that for at least one chromosome, there is only one copy of a chromosome present in the set of chromosomes of a given subject (e.g., fetal set of chromosomes). As almost all full monosomies are lethal (except, e.g., mammal (human) monosomy for X where also no Y is present (X0; "Turner syndrome"), the term "monosomy" as used herein also comprises "partial monosomy" such as, e.g. (for mammals, humans), partial monosomy for chromosome 1 ("1p36 deletion syndrome") or chromosome 5 ("Cri-du-chat syndrome"), where only parts or segments of the respective chromosomes are missing (also known and referred to herein as structural chromosomal aberration or structural chromosomal disorder). That is, the term "monosomy" as used herein also comprises "partial monosomy" where only one copy of a particular chromosome (autosome or sex chromosome) is present or part or a segment of a particular chromosome is missing in a cell of a given subject (e.g., fetus, particularly human fetus). For example, in context with the present invention, "monosomy" may comprise a monosomy for example (particularly in case the subject is mammal, e.g., human) for one or more of chromosomes 21, 18, 13, 1 or 5 or parts thereof, where only one copy of said chromosome is present or part of one of said chromosomes is missing (also "partial monosomy") in a cell or all cells of the subject (fetus, e.g., human fetus), or a X0 monosomy where only one sex chromosome (X) is present. In one embodiment of the present invention "monosomy" means only one copy of chromosome 21, 18, 13, or missing part or segment of chromosome 1 ("1p36 deletion syndrome", (partial) monosomy 1") or chromosome 5 ("Cri-du-chat syndrome", "(partial) monosomy 5"), or only one copy of X (and no Y) ("Turner syndrome", "monosomy X", X0). In a particular embodiment of the present invention, the fetal monosomy status is (partial) monosomy 21, 18, 13, 5 or X, or particularly monosomy 21, 18, or 13 (or X).

In one embodiment of the present invention, relating to the NIPT method for determining whether or not a sample obtained from a pregnant subject, preferably a human subject is qualified for determining the fetal trisomy status of a chromosome, $f_{LoD}^{T}$ may be given by the following formula

$$f_{LoD}^{T} = \left( \frac{t_{max}^{E}}{t_{min}^{E}} - 1 \right) * 2. \qquad (2)$$

In one embodiment of the present invention, relating to the NIPT method for determining whether or not a sample obtained from a pregnant subject, preferably a human subject is qualified for determining the fetal trisomy status of a chromosome, if $t > t_{max}^{E}$ , then the fetal trisomy status is determined as trisomic (e.g., trisomy 13, 18, 21 or X).

In one embodiment of the present invention, relating to the NIPT method for determining whether or not a sample obtained from a pregnant subject, preferably a human subject is qualified for determining the fetal trisomy status of a chromosome, the read representation of a chromosome t may be replaced by the z-score z

$$z = \frac{t - \mu^E}{\sigma^E},$$

wherein $\mu^E$ is an estimate of the location of the distribution of the read representation of the chromosome in a population that is euploid in the chromosome, $\sigma^E$ is an estimate of the scale of the distribution of the read representation of the chromosome in a population that is euploid in the chromosome, comprising determining whether

(a)

$$z \le f_{LoD,z}^T \qquad\qquad (5a)$$

(b)

$$z \ge k_f^T * z_{min}^E + \left(k_f^T - 1\right) * \mu^E / \sigma^E \qquad\qquad (5b)$$

(c)

$$z \le z_{max}^E \qquad\qquad (5c)$$

wherein

$$f_{LoD,z}^T = \frac{2 * (z_{max}^E - z_{min}^E)}{z_{min}^E + \mu^E / \sigma^E}$$

$$k_f^T = (1 + 0.5 * f)$$

$z_{min}^E$ is the minimal z-score in a set of samples that are euploid for the chromosome, and

$z_{max}^E$ is the maximal z-score in a set of samples that are euploid for the chromosome;
wherein, if all of (a) to (c) are fulfilled, said sample is not qualified for determining the fetal trisomy status of the chromosome, or
wherein, if any one of (a) to (c) is not fulfilled, said sample is qualified for determining the fetal trisomy status of the chromosome. If $z > z_{max}^E$, then the trisomy status may be determined as trisomic (e.g., trisomy 18, 13, 21, X).

In one embodiment of the present invention, relating to the NIPT method for determining whether or not a sample obtained from a pregnant subject, preferably a human subject is qualified for determining the fetal monosomy status of a chromosome, the fetal monosomy status is monosomy 21, 13, 18, 1, 5, or X.

In one embodiment of the present invention, relating to the NIPT method for determining whether or not a sample obtained from a pregnant subject, preferably a human subject is qualified for determining the fetal monosomy status of a chromosome, $f_{LoD}^M$ may be given by the following formula

$$f_{LoD}^M = \left(1 - \frac{t_{min}^E}{t_{max}^E}\right) * 2. \qquad (4)$$

In one embodiment of the present invention, relating to the NIPT method for determining whether or not a sample obtained from a pregnant subject, preferably a human subject is qualified for determining the fetal monosomy status of a chromosome, if $t < t_{min}^E$, then the monosomy status is determined as monosomic (e.g., monosomy 21, 13, 18, X, or partial monosomy 1 or 5).

In one embodiment of the present invention, relating to the NIPT method for determining whether or not a sample obtained from a pregnant subject, preferably a human subject is qualified for determining the fetal monosomy status of a chromosome, the read representation of the chromosome t may be replaced by the z-score z

$$z = \frac{t - \mu^E}{\sigma^E},$$

wherein $\mu^E$ is an estimate of the location of the distribution of the read representation of the chromosome in a population that is euploid in the chromosome, $\sigma^E$ is an estimate of the scale of the distribution of the read representation of the chromosome in a population that is euploid in the chromosome, comprising determining whether

(a)

$$z \leq f_{LoD,z}^M$$

(b)

$$z \geq k_f^M * z_{min}^E + \left(k_f^M - 1\right) * \mu^E / \sigma^E$$

(c)

$$z \leq z_{max}^E$$

wherein

$$f_{LoD,z}^M = \frac{2 * (z_{max}^E - z_{min}^E)}{z_{max}^E + \mu^E / \sigma^E}$$

$$k_f^M = (1 - 0.5 * f)$$

$z_{min}^E$ is the minimal z-score in a set of samples that are euploid for the chromosome, and

$z_{max}^E$ is the maximal z-score in a set of samples that are euploid for the chromosome;

wherein, if all of (a) to (c) are fulfilled, said sample is not qualified for determining the fetal monosomy status of the chromosome, or

wherein, if any one of (a) to (c) is not fulfilled, said sample is qualified for determining the fetal monosomy status of the chromosome. If $z < z_{min}^E$ , then the monosomy status is determined as monosomic (e.g., monosomy 21, 13, 18, 1, 5, or X).

In one embodiment of the present invention, if a sample is qualified for determining fetal trisomy or is qualified for determing fetal monosomy and the read representation of a chromosome $t$ is such that $t > t_{min}^E$ and $t < t_{max}^E$ , then the ploidy status is determined as euploid in the chromosome.

In one embodiment of the present invention, if a sample is qualified for determining fetal trisomy or is qualified for determining fetal monosomy and the read representation of a chromosome t is replaced by the z-score z as described herein, and the z-score z is such that $z > z_{min}^E$ and $z < z_{max}^E$ , then the ploidy status is determined as euploid in the chromosome.

In one embodiment of the present invention, the read representation of a chromosome t may be replaced by the normalized read counts over a subset of disjoint subregions of the chromosome.

The present invention further relates to a NIPT method for determining the limit of detection (LoD) $f_{LoD}^T$ for trisomy on a chromosome within the read-counting approach to the non-invasive prenatal testing, comprising determining the minimal value $t_{min}^E$ of the read representation of the chromosome in a set of euploid samples and the maximal value $t_{max}^E$ of the read representation of the chromosome in a set of euploid samples, and determining $f_{LoD}^T$ according to the formula

$$f_{LoD}^T = \left(\frac{t_{max}^E}{t_{min}^E} - 1\right) * 2.$$

The present invention further relates to a NIPT method for determining the limit of detection (LoD) $f_{LoD}^M$ for monosomy of a chromosome within the read-counting approach to the non-invasive prenatal testing, comprising determining the minimal value $t_{min}^E$ of the read representation of the chromosome in a set of euploid samples and the maximal value $t_{max}^E$ of the read representation of the chromosome in a set of euploid samples, and determining $f_{LoD}^M$ according to the formula

$$f_{LoD}^M = \left(1 - \frac{t_{min}^E}{t_{max}^E}\right) * 2.$$

As mentioned above, the present invention provides a NIPT method for the fetal aneuploidy status determination in the non-invasive prenatal testing which takes into account both fetal fraction $f$ and read representation of a chromosome $t$.

In one embodiment, the NIPT method described and provided by the present invention includes determination whether the pair ($f$, $t$) belongs to the Triangle of Uncertainty for Trisomy (TUT) for a chromosome or to the Triangle of Uncertainty for Monosomy (TUM) for a chromosome. TUT and TUM are based on the basic correspondence between the read representation of a chromosome t and the composition of the maternal circulating cell free DNA (cfDNA). If the composition of cfDNA is such that for a trisomic pregnancy the triple chromosomes of the fetus comprise f percent, and the couple of maternal chromosomes comprise 1-f percent, then the resulting read representation of a chromosome $t$ is

$$t = (1 - f) * t^E + 1.5 * f * t^E, \qquad (1)$$

where $t^E$ is the read representation of the chromosome in the euploid mother. Due to the technical variabality in Massive Parallel Sequencing (MPS), the values of the read representation of the chromosome of euploid mothers may vary and lay in a range $[t_{min}^E, t_{max}^E]$. This variability is transferred through (1) into a variability of the trisomic samples. This is the reason why a trisomic sample may be such that its read representation of the chromosome t may lay in the euploid range $[t_{min}^E, t_{max}^E]$, for certain values of fetal fraction f. Hence, for certain values ($f$, $t$), it may not be determinable whether the fetus is euploid or trisomic in the chromosome. In context with this embodiment of the present invention, these values form the TUT. The same applies *mutatis mutandis* for monosomy and TUM, where the resulting read representation of a chromosome t of monosomic pregnancy is calculated by formula (1'):

$$t = (1 - f)t^E + 0.5 \, f \, t^E. \qquad (1')$$

The Triangle of Uncertainty for Trisomy (TUT) for a chromosome comprises all the pairs ($f$, $t$) of the values of fetal fraction $f$ and the read representation t of the chromosome, such that:

$$f \leq f_{LoD}^T \qquad (1a)$$

$$t \geq (1 + 0.5 * f) * t_{min}^E \qquad (1b)$$

$$t \leq t_{max}^{E} \tag{1c}$$

t is the read representation of the chromosome,

$f_{LoD}^{T}$ is the limit of detection (LoD) for trisomy of the chromosome,

$t_{min}^{E}$ is the minimal value of the read representation of the chromosome in a set of euploid samples, and

$t_{max}^{E}$ is the maximal value of the read representation of the chromosome in a set of euploid samples.

In context with the present invention, the LoD for trisomy of the chromosome may be determined by the following formula:

$$f_{LoD}^{T} = \left( \frac{t_{max}^{E}}{t_{min}^{E}} - 1 \right) * 2. \tag{2}$$

In an embodiment of the present invention, the Triangle of Uncertainty for Monosomy (TUM) for a chromosome comprises all the pairs *(f, t)* of the values of fetal fraction f and the read representation t of the chromosome, such that:

$$f \leq f_{LoD}^{M} \tag{3a}$$

$$t \leq (1 - 0.5 * f) * t_{max}^{E} \tag{3b}$$

$$t \geq t_{min}^{E} \tag{3c}$$

t is the read representation of the chromosome,

$f_{LoD}^{M}$ is the limit of detection (LoD) for a monosomy of the chromosome,

$t_{min}^{E}$ is the minimal value of the read representation of the chromosome in a set of euploid samples, and

$t_{max}^{E}$ is the maximal value of the read representation of the chromosome in a set of euploid samples.

In context with the present invention, the LoD for monosomy of the chromosome may be determined by the following formula:

$$f_{LoD}^{M} = \left( 1 - \frac{t_{min}^{E}}{t_{max}^{E}} \right) * 2 \tag{4}$$

The read representation of a chromosome can be obtained by means of counting reads from the Whole Genome MPS as described herein, which are mapped to a reference human genome, with a possible subsequent filtering, preprocessing (e.g., by the GC correction), normalization (e.g., by the total number of reads in autosomes). The read representation of a chromosome may involve the entire chromosome or its disjoint segments. An estimate of the minimal and maximal values of the read representation of a chromosome in an euploid population may be obtained from samples from such a population. The fetal fraction can be determined by methods known in the art and described herein, for instance by various *in silico* algorithms, such as seqFF.

In context with the present invention, if there is the following information available for a particular sample (e.g., pregnant subject or fetus; preferably mammal, more preferably human):

i) the read representation t of a chromosome or its disjoint segments,
ii) fetal fraction (*f*),
iii) the minimal $t_{min}^{E}$ and maximal values $t_{max}^{E}$ of the read representation t of the chromosome in samples with confirmed euploid status,

then the aneuploidy status of the sample can be determined using the following decision tree:
*If (f, t) are such, that:*

a) $(t > t_{max}^E)$ , then the sample is considered trisomic of the chromosome,

b) $(t < t_{min}^E)$ , then the sample is considered monosomic of the chromosome,

c) *(f, t)* belong to the TUT for the chromosome, then it cannot be decided whether the sample is trisomic or euploid, i.e. the sample is not qualified for determination of the fetal trisomy status of the chromosome,

d) *(f, t)* belong to the TUM for the chromosome, then it cannot be decided whether the sample is monosomic or euploid, i.e. the sample is not qualified for determination of the fetal monosomy status of the chromosome,

e) otherwise, the sample is considered euploid for the chromosome.

In one embodiment of the present invention, the TUT can be equivalently defined in terms of the z-score in place of the read representation of a chromosome t. The z-score is just a scaled form of the read representation t of the chromosome. The Z-score Triangle of Uncertainty for Trisomy (zTUT) for a chromosome is defined by the following three requirements:

$$f \leq f_{LoD,z}^T \tag{5a}$$

$$z \geq k_f * z_{min}^E + (k_f - 1) * \mu^E/\sigma^E \tag{5b}$$

$$z \leq z_{max}^E \tag{5c}$$

z is the z-score of a sample, which is given as

$$z = (t - \mu^E)/\sigma^E$$

where t is the read representation of the chromosome, $\mu^E$ is an estimate of the location of the distribution of the read representation of the chromosome (e.g., the sample average or the sample median) in a population euploid for the chromosome, and $\sigma^E$ is an estimate of the scale of the distribution of the read representation of the chromosome (e.g., the standard deviation or the interquartile range) in a population euploid for the chromosome. Also,

$$k_f = 1 + 0.5 * f,$$

and the z-score LoD for trisomy on the chromosome $f_{LoD,z}^T$ may be

$$f_{LoD,z}^T = \frac{2 * (z_{max}^E - z_{min}^E)}{z_{min}^E + \mu^E/\sigma^E}$$

where $z_{min}^E$ is the minimal value of the z-score for the chromosome in euploid samples, and $z_{max}^E$ is the maximal value of the z-score for the chromosome in euploid samples.

In another embodiment of the present invention, the TUM can equivalently be formulated in terms of the z-score and the Z-score Triangle of Uncertainty for Monosomy (zTUM) for a chromosome be obtained.

In this context, the above-mentioned decision tree can equivalently be determined by means of zTUT and zTUM. If the z-score *z* for a chromosome and fetal fraction f are such that:

a) ( $z > z_{max}^E$ ), then the sample is considered trisomic of the chromosome,

b) ( $z < z_{min}^E$ ), then the sample is considered monosomic of the chromosome,

c) *(f,* z) belong to the zTNT for the chromosome, the the sample is either euploid or trisomic on the chromosome, and hence its status cannot be called, i.e. the sample is not qualified for determination of the fetal trisomy status of the chromosome,

d) (f, z) belong to the zTNM for the chromosome, then the sample is either euploid or monosomic on the chromosome, and hence its status cannot be called, i.e. the sample is not qualified for determination of the fetal monosomy status of the chromosome,

e) otherwise, the sample is considered euploid for the chromosome.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention. Also, the term "at least" preceding a particular amount or number also comprises exactly this amount or number, i.e. it may also serve as specification of a particular amount or number.

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

It should be understood that this invention is not limited to the particular methodology, protocols, and reagents, etc., described herein and as such can vary. The terminology used herein is for the purpose of describing particular embodiments only, and is not intended to limit the scope of the present invention, which is defined solely by the claims.

BRIEF DESCRIPTION OF THE FIGURES

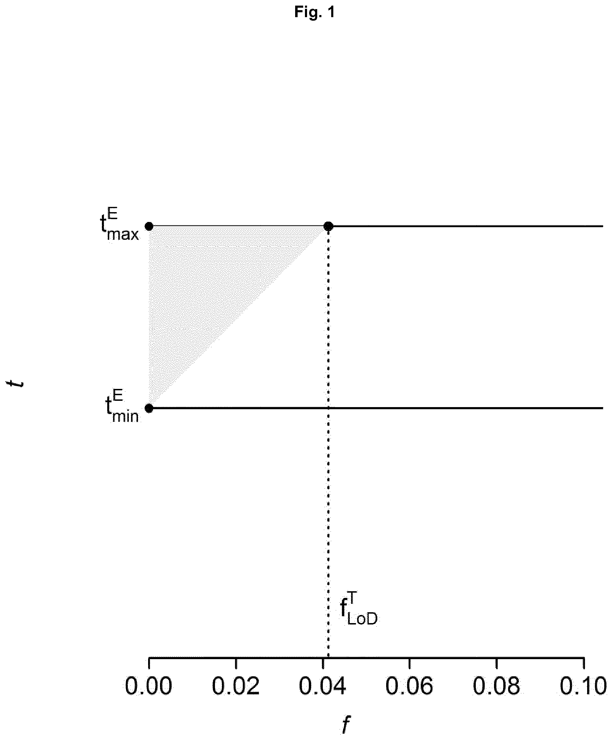

Figure 1: Schematic depiction of the Triangle of Uncertainty for Trisomy. The vertices of the TUT are: (0, $t^E_{min}$), (0, $t^E_{max}$), ($f^T_{LoD}$, $t^E_{max}$).

Figure 2: The Z-score Triangle of Uncertainty for Trisomy. The square with the number '1' denote the sample analyzed in Example 1.

Figure 3: The Z-score Triangle of Uncertainty for Trisomy. The square and the number '2' denote the sample from Example 2.

Figure 4: The Z--score Triangle of Uncertainty for Trisomy. The square and the number '3' denote the sample from Example 3.

Figure 5: Triangle of uncertainty for trisomy, monosomy, and the plygon of uncertainty for aneuploidy.

The formula (1) defines a line in the (f,t) plane, referred to as the trisomy line, with the intercept $t^E$ and the slope $0.5t^E$. The trisomy line is exhibited on Fig. 5a for the two extreme cases, $t^E_{min}$, $t^E_{max}$; see the diagonal dashed lines in Fig. 5a. Every trisomic pregnancy with (f,t) that lays above or on the trisomy line for $t^E_{min}$ and has the value of t in $[t^E_{min}, t^E_{max}]$ cannot be distinguished from an euploid pregnancy. The set of such 'no call' pairs (f,t) forms the gray triangle on Fig. 5a. The vertices of the triangle of uncertainty for trisomy (TUT), $\Delta^T$, are (0, $t^E_{min}$), (0, $t^E_{max}$), and (

$f_{LoD}^{T}$, $t^{T}(f_{LoD}^{T})$ ), where $f_{LoD}^{T}$ is the LoD for trisomy. The trisomic LoD $f_{LoD}^{T}$ is obtained from the requirement that it solves $t(f, t_{min}^{E}) = t_{max}^{E}$. The triangle of uncertainty for trisomy, $\Delta^{T}$, comprises all such pairs *(f,t)* of values of fetal fraction f and the read representation of a chromosome t as shown in formulae (1), (1a), (1b) and (1c). LoD for trisomy is calculated as shown in formula (2). $f_{LoD}^{T}$ can be decreased by decreasing the range of euploid read representation of a chromosomes (i.e., $t_{max}^{E} - t_{min}^{E}$ ) and/or by increasing $t_{min}^{E}$ .

An analogous reasoning applies for a monosomy. For a monosomy, the basic correspondence formula is shown in (1'). The triangle of uncertainty for monosomy (TUM), $\Delta^{M}$ (see Fig. 5b), is formed by all such pairs (f,t) of values of fetal fraction f and the read representation of a chromosome t as shown in formulae (3a), (3b) and (3c). The LoD for monosomy is shown in formula (4). Union of the triangles $\Delta^{T}$, $\Delta^{M}$, forms the polygon of uncertainty for aneuploidy, which resembles a stylized letter 'K'; see Fig. 5c. The polygon of uncertainty has five vertices: *A* is (0, $t_{min}^{E}$ ), B is (0, $t_{max}^{E}$ ), C is $(f_{LoD}^{T}, t^{T}(f_{LoD}^{T}))$ , *E* is $(f_{LoD}^{M}, t^{M}(f_{LoD}^{M}))$ , and D lays on the intersection of the *AC, BE* segments. If a sample is such that (f,t) falls into the polygon of uncertainty, then it cannot be determined whether the sample is euploid or aneuploid (i.e., trisomic, monosomic). In particular, if (f,t) are in the triangle *BCD* at Fig. 5c, then the sample can be trisomic or euploid. If (f,t) are in the triangle *ADE* then the sample can be either monosomic or euploid in the chromosome. If (f,t) lay in the triangle *ABD,* then the sample can be either trisomic, monosomic or euploid.

EXAMPLES

**Example 1**

Step 1) The Whole Genome Massive Parallel Sequencing, bioinformatic processing, reads mapping, filtering, binning, read representation of a chromosome

cfDNA from a blood sample from a pregnant woman was sequenced by MPS. The resulting reads were mapped to the human genome hg19 by the algorithm bowtie2.
Duplicate and low-quality reads were afterwards filtered out. Then the reads were binned into 50 kilo base pair bins. The total number of reads in a subset of bins was normalized by the total read counts in all autosomes.
This way the read representation of a chromosome was obtained for chromosomes 13, 18, 21. The read representation of the chromosome 21 was t = 0.007476537.

Step 2) Determination of the minimal and maximal values of the read representation of the chromosome 21 in euploid pregnancies

In a set of 99 samples with confirmed euploidy on chromosome 21 the minimal value of the read representation of the chromosome 21, in the same subset of bins as used in Step 1, was equal $t_{min}^{E} = 0.007282826$ . The maximal value was $t_{max}^{E} = 0.007416512$ . This, by the Formula (2) gives the LoD for trisomy 21 to be $f_{LoD}^{T21} = 0.03671255$ , i.e., 3.67%.

Step 3) Fetal fraction determination

Fetal fraction for the pregnancy was estimated by the method seqFF, developed by Sequenom Inc. (cf. Kim et al., Prenat. Diagn. (2015), 35: 810-815). The value of fetal fraction was $f = 0.02899$ , i.e., 2.9%.

Step 4) Fetal aneuploidy status determination

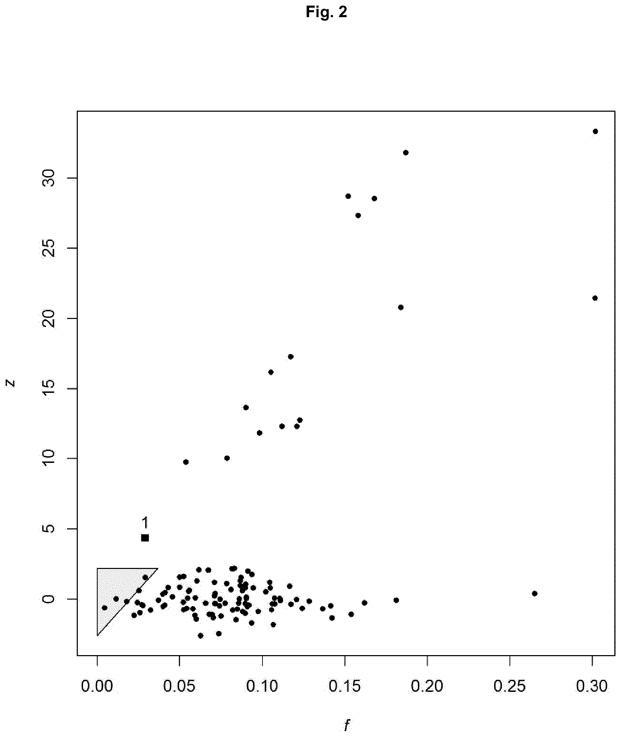

The value of fetal fraction f is lower than the limit of detection $f_{LoD}^{T21}$ , since 0.02899 < 0.03671255. Hence, in the conventional NIPT this sample would be reported as 'no call'.
When the decision tree, stated above, is used, then we see that the analyzed sample is the case a), since the read representation of the chromosome 21 is t = 0.007476537 which is greater than $t_{max}^{E} = 0.007416512$ . Which means that the sample is called/reported as trisomic in the chromosome 21. The same conclusion is obtained with the decision tree that utilizes the z-score. Indeed, since $\mu^{E21} = 0.007355081$ and $\sigma^{E21} = 2.787415e - 05$, the z-score of the sample is 4.357. The maximal value of the z-score for chromosome 21 in samples that are euploid on chromosome 21 is 2.203878, hence the sample satisfies the first condition $z > z_{max}^{E}$ of the decision tree, and hence the aneuploidy status of the sample is trisomic in chromosome 21; see Figure 2 and the point denoted by ,1'.

Let us note that the sample was later on confirmed to be trisomic.

Summary. Example 1 demonstrates the case when the conventional NIPT fails to discover the chromosome abnormality, due to the low fetal fraction. The conventional NIPT ignores the read representation of a chromosome. The present invention takes both fetal fraction and read representation of a chromosome into account via the decision tree that utilizes the Triangle of Uncertainty for Trisomy, the Triangle of Uncertainty for Monosomy. In this particular sample the conventional NIPT would fail to detect trisomy 21. The present invention would recognize the trisomic state for chromosome 21 of the sample.

## Example 2

For another sample, the read representation of the chromosome 21 was t = 0.007333882, fetal fraction was estimated to be $f = 0.0323785$. Since the fetal fraction is below LoD $f_{LoD}^{T21}$, the conventional NIPT would fail to determine the aneuploidy status.

When the above-described decision tree is utilized, we find out that the sample belongs to the case e), since none of the requirements a)-d) is satisfied. Hence, the decision tree calls the sample euploid in chromosome 21.

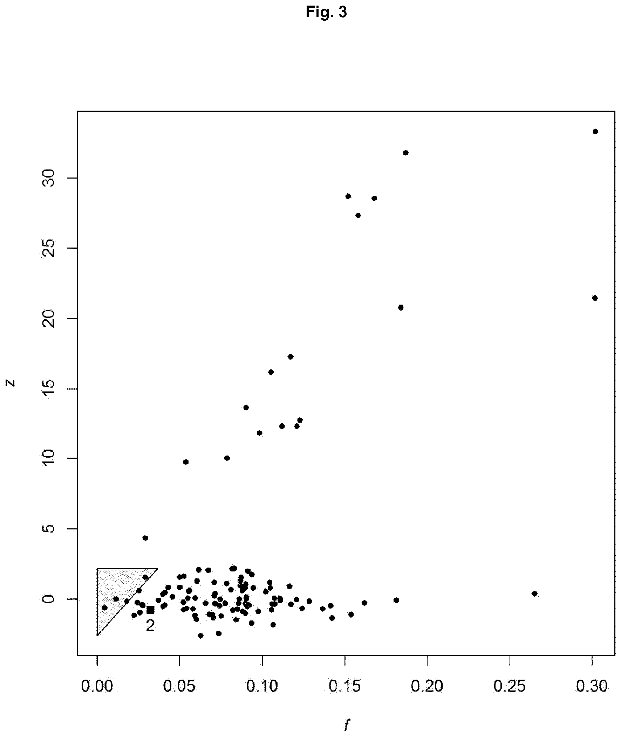

The same conclusion is drawn by the equivalent, z-score version of the decision tree. The z-score of the sample for chromosome 21 is z = -0.760519, $k_f$=1.016189, $z_{min}^{E} = -2.592162$, hence the sample does not belong to the zTUT; see Figure 3, where the sample is denoted by '2'. In a similar way it can be checked that the sample does not belong to the zTUM. The cases a) and b) are clearly excluded, as well. Hence the sample is euploid in chromosome 21. Let us note that the sample was confirmed to be euploid in chromosome 21.

Summary. Example 2 demonstrates the case where the conventional NIPT cannot determine the fetal aneuploidy status due to the low fetal fraction. The conventional NIPT, however, ignores the information about the read representation of a chromosome. The present invention takes into account both fetal fraction and read representation of a chromosome via the decision tree, that utilizes the TUT, TUM, or equivalently the zTUT, zTUM. The conventional NIPT would not recognize that the sample is euploid on chromosome 21, whereas the present invention would recognize the sample as euploid.

## Example 3

For yet another sample, the read representation of a chromosome was t = 0.007355957, fetal fraction was determined to be f = 0.01138656. Since f is below $f_{LoD}^{T21}$, the conventional NIPT could not determine the aneuploidy status of the sample in the chromosome 21.

If the above-stated decision tree is used, then we see that the sample is the case c), since

$$f \leq f_{LoD}^{T} \qquad [0.01138656 \leq 0.03671255], \text{ and}$$

$$t \geq (1 + 0.5 * f) * t_{min}^{E}, \qquad [0.007355957 \geq 0.00732429], \text{ and}$$

$$t \leq t_{max}^{E}, \qquad [0.007355957 \leq 0.007416512].$$

Hence, the present invention cannot determine the aneuploidy status of the sample.

The same conclusion is obtained by the equivalent z-score decision tree. Indeed, since the z-score of the sample is z = 0.03144595, $f_{LoD,z}^{T} = 0.03671255$, and $k_f$ = 1.005693, thus

$$f \leq f_{LoD,z}^{T} \qquad [0.01138656 < 0.03671255]$$

(continued)

$$z \geq k_f * z_{min}^E + \left(k_f - 1\right) * \frac{\mu^E}{\sigma^E} \qquad [0.03144595 \geq -1.104649]$$

$$z \leq z_{max}^E \qquad [0.03144595 < 2.203878]$$

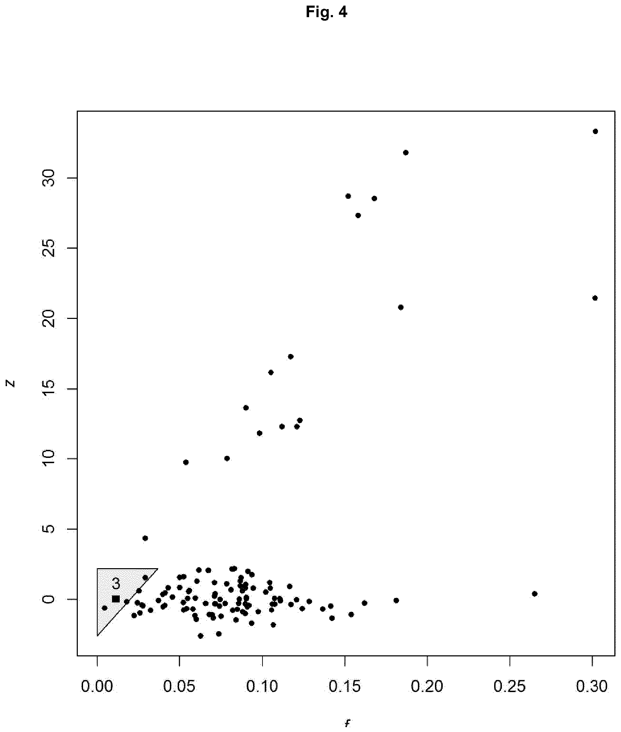

hence the sample belongs to the zTUT; see Figure 4 and there the point denoted '3'. The analyzed sample belongs to the the zTUT and hence it cannot be determined whether it is euploid or trisomic for chromosome 21. Let us mention that the sample was later on determined to be euploid for the chromosome 21.

Summary: Example 3 demonstrates the case where neither the conventional NIPT nor the present invention can determine the aneuploidy status of a chromosome. The conventional NIPT ignores the information on the read representation of a chromosome. This is the reason why in the conventional NIPT the number of 'no call' samples is (much) higher than in the present invention.

In the analyzed set of samples the conventional NIPT failed to call aneuploidy status of chromosome 21 for 12 samples whereas the present invention failed to call the aneuploidy status in 4 samples, only. The marked increase in the detection rate is due to the fact that the present invention takes into account both the read representation of a chromosome and fetal fraction, via the decision tree that is based on the TUT, TUM, or equivalently, the zTUT, zTUM.

Among the samples missed by the conventional NIPT, there was a trisomy 21 sample that was detected by the present invention. The present invention is thus capable of increasing the detection rate of NIPT, while retaining its high sensitivity and specificity.

### Example 4

Patient cohort

Maternal blood samples were obtained from 116 subjects at the Department of Obstetrics and Gynaecology of Jessenius Faculty of Medicine in Martin (Slovakia) based on the following criteria: minimal maternal age is 18 years, minimal gestational age is 9 gestational weeks (gw), singleton pregnancy. The descriptive summary statistics, i.e., the lowest value, lower quartile Q1, median, mean, upper quartile $Q_3$, and the highest value, are in Table 1.

Table 1: Summary statistics for the gestational weeks (gw), maternal age, and maternal weight

|  | min | $Q_1$ | median | mean | $Q_3$ | max |
|---|---|---|---|---|---|---|
| gw | 9.00 | 12.00 | 15.00 | 15.47 | 18.00 | 26.00 |
| maternal age [yrs] | 25.0 | 30.0 | 33.5 | 33.4 | 36.0 | 44.0 |
| maternal weight [kg] | 49.50 | 61.00 | 67.00 | 70.14 | 76.00 | 110.00 |

Aneuploidy and fetal sex were determined by invasive prenatal diagnostic methods (CVS or amniocentesis). This clinical data were blinded to all participants of subsequent processing, until the determination of the outcomes of NIPT. Among 116 patients there were 17 trisomies 21 (T21), 2 trisomies 18 (T18), and 6 trisomies 13 (T13).

Sample collection and cfDNA isolation

Peripheral blood samples for NIPT testing were taken from pregnant women prior to invasive diagnostic assessment. 10 ml of venous maternal blood from each woman was collected into EDTA tubes and then stored at 4-8 °C. The plasma was separated from samples within 24 hours after collection. The plasma was extracted by two step centrifugation at 3000 rpm and 14,500 rpm each at 4 °C, for 10 min and immediately stored at —80 °C in barcoded tubes, until further processing. The cell-free DNA was isolated from 1 ml of maternal plasma using optimized protocol of DSP Virus Kit (Qiagen, Hilden, Germany). The protocol was optimized for 1 ml of plasma input, elution time was 30 min, and the final elution volume was 68 |jl.

Library preparation and sequencing

After DNA isolation, the quality and quantity of cfDNA was monitored as each sample had to pass a quality control. The TruSeq Nano DNA Library Preparation Kit (Illumina, USA) was used to prepare DNA libraries. Because of the naturally occurring fragmentation of cfDNA, it was continued with repair ends and selected library size. Optimized ratios of Sample Purification Beads 0,7 X and 1,7 X were used which allowed to obtain libraries with the average length 295-300 bp. 18 to 24 libraries were equimolarly pooled and the mixture was subsequently diluted to 2 nM. It was sequenced with 51-cycles in the single read mode, using Illumina HiSeq 1500 platform (Illumina, USA).

Bioinformatic processing

Each run resulted in 18-20 million reads per sample. All sequencing data were demultiplexed and then mapped to the human reference genome (hg19, NCBI build 37) using Bowtie2 (Langmead et al., Nat. Methods 9, 357-359 (2012)). Unmapped, duplicate, low quality reads were filtered out by the Bioconductor (Huber et al., Nat. Methods 12, 115-121 (2015). URL http://dx.doi.org/10.1038/nmeth.3252. DOI 10.1038/nmeth.3252) library Rsamtools (Morgan et al., Rsamtools: Binary alignment (BAM), FASTA, variant call (BCF), and tabix file import. URL http://bioconductor.org/packages/release/bioc/html/Rsamtools.html. R package version 1.18.3). Reads were then aggregated over non-overlapping 50 kbp bins. Afterwards, the resulting chromosomal profiles were subjected to a two different normalization methods. **Method A** is the normalization according to Jensen et al., PloS One 8, e57381 (2013), as implemented in Kim et al., Prenat. Diagn. 35, 810-815 (2015). It involves chromosomal subset selection to minimize variability and the GC-bias correction.

**Method B** normalizes the binned read counts by the total read counts over all autosomes and then computes read representation of a chromosome in a chromosomal subset, selected by an in house method. Selection of the subregions has not assumed information about fetal fraction, thus avoiding circularity. Moreover, the subregions selection was cross-validated.

Fetal fraction estimation

Fetal fraction was estimated by the seqFF method (Kim et al., Prenat. Diagn. 35, 810-815 (2015)). Among 116 samples, there were 15 samples with fetal fraction below the conventional 4% cutoff, with the maternal weight in the range of 89-110 kg. To assess its performance, the seqFF was compared with the $\gamma$-chromosome method (Hudecova et al., PloS One 9, e88484 (2014); Chiu et al., BMJ 342, c7401 (2011)), denoted ffY, on a subset of 60 pregnancies bearing male fetuses. The average of normalized read counts for chromosome Y in 11 males, and the average representation of chromosome Y in the 56 female-carrying pregnancies were used for obtaining ffY.

The Spearman correlation of seqFF and ffY was 0.92. The median difference between the two methods was 0.3%. For 41 of the 60 pregnancies, seqFF produced lower estimate than ffY. The ffY method has found fetal fraction below the conventional 4% threshold in 4 pregnancies, whereas seqFF reported 11 pregnancies with fetal fraction below 4%. The set included the 4 pregnancies found by ffY.

*seqFFY method for fetal fraction estimation*

Though there are systematic and substantive differences among several methods for fetal fraction determination in male bearing pregnancies (Grendar et al., Prenat. Diagn. (2017), DOI 10.1002/pd.5151; van Beek et al., Prenat. Diagn. 37, 769-773 (2017). DOI 10.1002/pd.5079), the ffY method is considered reliable (Peng et al., Int. J. Mol. Sci. 18, 453 (2017)). In the latter reference, it was also noted that seqFF tends to underestimate fetal fraction at the low end of values. To mitigate the underestimation, the present inventors performed a modification of seqFF, denoted seqFFY, that borrows strength from both of the methods. For male carrying pregnancies seqFFY is just the ffY method, as it may be even more precise than seqFF. For female carrying pregnancies, ffY is not applicable as already mentioned above. However, it is possible to calibrate ffY to seqFF, and use the calibration curve to produce an estimate for a female carrying pregnancy, by predicting its ffY value based on its seqFF value. The calibration curve can be estimated by any of the regression fitting methods; the cross-validated smoothing spline (R Core Team. R: A Language and Environment for Statistical Computing. R Foundation for Statistical Computing, Vienna, Austria (2015). URL https://www.R-project.org/) was used to obtain the calibration curve.

Results

The Methods A, B involved different procedures for binned reads processing. Both they were used in conjunction with the seqFF method for fetal fraction estimation. The Method C utilized the same preprocessing pipeline as the Method B; however seqFF was replaced by seqFFY. The LoD for trisomy, monosomy, was estimated by formulae (2) and (4), respectively.

*Method A*

*Chromosome 21*

The LoD for trisomy 21 was 4.29%. Among 116 samples, 17 have fetal fraction below the LoD. Thus, the no-call rate for trisomy 21 due to the low fetal fraction is 14.66%. Among the 17 samples that would be missed by the conventional NIPT there was 1 sample with trisomy 21.

When the read representation of the chromosome 21 was taken into account and only the samples in $\Delta^T$ were considered undecidable, then the no call rate drops to 3.45%, corresponding to 4 uncertain cases. The uncertain cases were euploid. The trisomic sample that would be missed by the conventional approach was detected by the method of the present invention, and correctly reported as trisomic.

*Chromosome 18*

For trisomy 18, the LoD was 5.96%. There were 32 samples with fetal fraction below the LoD, resulting in enormous 27.59% no call rate of the conventional approach. When the read representation of the chromosome 18 was taken into account via $\Delta^T$, the no call rate decreased to 9.48% (i.e., 11 samples).

*Chromosome 13*

For trisomy 13, the LoD was 5.32%. There were 24 samples with fetal fraction below the LoD, resulting in 20.69% no call rate of the conventional approach. When the read representation of the chromosome 13 was taken into account through $\Delta^{T,}$ the no call rate decreased to 6% (i.e., 7 samples).

*Method B*

*Chromosome 21*

The LoD for trisomy 21 was 3.67%. There were 12 samples with fetal fraction below the LoD. Thus, the no-call rate for trisomy 21 due to the low fetal fraction was 10.35%. Among the 12 samples that would be missed by the conventional NIPT method, there was 1 sample with trisomy 21.

When the read representation of the chromosome 21 was taken into account via $\Delta^T$, then the no call rate decreased to 3.45%, corresponding to 4 uncertain cases. The uncertain cases were euploid. The trisomic sample that would be missed by the conventional NIPT was detected by the method of the present invention, and correctly reported as trisomic.

For the monosomy 21, the LoD was 3.61%, and there were 12 samples with f below the LoD. When the read representation of the chromosome 21 was taken into account via $\Delta^M$, the no call rate for the monosomy 21 dropped from 10.35% to 2.59%, corresponding to 3 undetermined samples.

*Chromosome 18*

The LoD for trisomy 18 was 3.71%. There were 13 samples with fetal fraction below the LoD. Thus, the no-call rate for the trisomy 18 due to the low fetal fraction was 11.21%.

When the read representation of the chromosome 18 was taken into account via $\Delta^T$, then the no call rate decreased to 4.31%, corresponding to 5 uncertain cases.

For the monosomy 18, the LoD was 3.64%, and there were 12 samples with f below the LoD. When the read representation of the chromosome 18 was taken into account via $\Delta^M$, the no call rate dropped from 10.35% to 1.72%, corresponding to 2 undetermined samples.

*Chromosome 13*

The LoD for trisomy 13 was 4.07%. There were 15 sample with fetal fraction below the LoD. Thus, the no-call rate for trisomy 13 due to the low fetal fraction was 12.93%. When the read representation of the chromosome was taken into account via $\Delta^T$, then the no call rate decreased to 5.17%, corresponding to 6 uncertain cases.

For the monosomy 13, the LoD was 3.99%, and there were 14 samples with f below the LoD. When the read representation of the chromosome 13 was taken into account via $\Delta^M$, the no call rate dropped from 12.07% to 2.59%, corresponding to 3 undetermined samples.

*Method C*

Method C used the same reads processing procedure as the Method B. This implies that the values of LoD, computed by formulae (2) and (4), are the same as those in the Method B. The two methods differ only in fetal fraction estimation procedure: Method C uses seqFFY whereas Method B uses seqFF as described herein. Consequently, the no call rate is different in Method C than in Method B.

*Chromosome 21*

Among 116 samples, 6 had fetal fraction below the LoD. Thus, the no call rate for trisomy 21 due to the low fetal fraction was 5.17%. Among the 6 samples that would be missed by the traditional method, there was 1 sample with trisomy 21.

When the read representation of the chromosome 21 was taken into account via $\Delta^T$, then the no call rate decreased to 0.86%, corresponding to 1 uncertain case. The uncertain case was euploid. The trisomic sample that would be missed by the conventional NIPT was detected by the method of the present invention, and correctly reported as trisomic.

For monosomy 21, there were 6 samples with f below the LoD. All the missed samples were euploid. When the read representation of the chromosome 21 was taken into account via $\Delta^M$, the no call rate dropped from 5.17% to 0%, corresponding to 0 undetermined samples. Polygon of uncertainty for aneuploidy in chromosome 21, obtained by overlaying $\Delta^T$ and $\Delta^M$, contained a single sample.

*Chromosome 18*

There were 6 samples with fetal fraction below the LoD. Thus, the no-call rate for trisomy 18 due to the low fetal fraction was 5.17%. When the read representation of the chromosome 18 was taken into account via $\Delta^T$, then the no call rate decreaseed to 0.86%, corresponding to 1 uncertain case. The uncertain case was euploid.

For monosomy 18, there were 6 samples with f below the LoD. When the read representation of the chromosome 18 was taken into account via $\Delta^M$, the no call rate dropped from 5.17% to 0%. The polygon of uncertainty for aneuploidy in chromosome 18, obtained by overlaying $\Delta^T$ and $\Delta^M$, contained a single sample.

*Chromosome 13*

Among 116 samples, 9 had fetal fraction below the LoD. Thus, the no call rate for trisomy 13 due to the low fetal fraction was 7.76%. When the read representation of the chromosome 13 was taken into account via $\Delta^T$, then the no call rate decreased to 0.86%, corresponding to 1 uncertain case. The case was euploid.

For monosomy 13, there were 6 samples with f below the LoD. When the read representation of the chromosome 13 was taken into account via $\Delta^M$, the no call rate dropped from 5.17% to 0%, corresponding to 0 undetermined samples.

The polygon of uncertainty for aneuploidy in chromosome 13, obtained by overlaying $\Delta^T$ and $\Delta^M$, contained a single sample.

Summary of results

Tthe method of the present invention is capable of substantive reduction of the no call rate of NIPT, relative to the conventional approach that ignores the information on read representation of a chromosome. This has been proven for both the Method A and Method B of read normalization, accompanied with the seqFF method as exemplary method for fetal fraction determination. The lowest no call rate was attained when the test was used in tandem with the Method C (see Table 2), which utilizes the seqFFY method for fetal fraction estimation. For Method C, the reduction of the no call rate was from the values 5%-8% of the conventional method to the values 0%-1% according to the method of the present invention. The single sample that was missed by the method of the present invention was euploid. Moreover, at chromosome 21, the inventive method has detected a trisomic case, that was missed by the conventional NIPT.

Table 2: Comparison of the conventional NIPT and the method of the present invention for Method C. LoD is the limit of detection obtained by formulae (2) and (4). The no call rate and the number of missed aneuploidies for the conventional NIPT and the method of the present invention are in the rightmost four columns. T=Trisomy; M=Monosomy

| chromosome | aneuploidy | LoD [%] | Conventional NIPT | | Method of the present invention | |
|---|---|---|---|---|---|---|
| | | | no call rate [%] | missed aneuploidies | no call rate [%] | missed aneuploidies |
| 21 | T | 3.67 | 5.17 | 1 | 0.86 | 0 |
| | M | 3.61 | 5.17 | 0 | 0 | 0 |
| 18 | T | 3.71 | 5.17 | 0 | 0.86 | 0 |
| | M | 3.64 | 5.17 | 0 | 0 | 0 |
| 13 | T | 4.07 | 7.76 | 0 | 0.86 | 0 |
| | M | 3.99 | 5.17 | 0 | 0 | 0 |

## Claims

1. A NIPT method for determining whether or not a sample obtained from a pregnant subject, preferably a human subject is qualified for determining the fetal trisomy status of a chromosome, comprising determining whether

   (a)

   $$f \leq f_{LoD}^{T}$$

   (b)

   $$t \geq (1 + 0.5 * f) * t_{min}^{E}$$

   (c)

   $$t \leq t_{max}^{E}$$

   wherein $f$ is the fetal fraction,
   t is the read representation of the chromosome,
   $f_{LoD}^{T}$ is the limit of detection (LoD) for trisomy of the chromosome,
   $t_{min}^{E}$ is the minimal value of the read representation of the chromosome in a set of euploid samples, and
   $t_{max}^{E}$ is the maximal value of the read representation of the chromosome in a set of euploid samples;
   wherein, if all of (a) to (c) are fulfilled, said sample is not qualified for determining the fetal trisomy status of the chromosome, or
   wherein, if any one of (a) to (c) is not fulfilled, said sample is qualified for determining the fetal trisomy status of the chromosome.

2. The method of claim 1, wherein the fetal trisomy status is trisomy 13, 18, 21 or triple X.

3. The method of claim 1 or 2, wherein $f_{LoD}^{T}$ is given by the following formula

$$f_{LoD}^{T} = \left( \frac{t_{max}^{E}}{t_{min}^{E}} - 1 \right) * 2.$$

**4.**

The method of any one of claims 1 to 3, wherein, if $t > t_{max}^E$ , then the fetal trisomy status is determined as trisomic for the chromosome.

**5.** The method of any one of claims 1 to 3, wherein the read representation of a chromosome t is replaced by the z-score z

$$z = \frac{t - \mu^E}{\sigma^E},$$

wherein $\mu^E$ is an estimate of the location of the distribution of the read representation of the chromosome in a population that is euploid in the chromosome, $\sigma^E$ is an estimate of the scale of the distribution of the read representation of the chromosome in a population that is euploid in the chromosome, comprising determining whether

(a)

$$z \leq f_{LoD,z}^T$$

(b)

$$z \geq k_f^T * z_{min}^E + \left(k_f^T - 1\right) * \mu^E / \sigma^E$$

(c)

$$z \leq z_{max}^E$$

wherein

$$f_{LoD,z}^T = \frac{2 * (z_{max}^E - z_{min}^E)}{z_{min}^E + \mu^E / \sigma^E}$$

$$k_f^T = (1 + 0.5 * f)$$

$z_{min}^E$ is the minimal z-score in a set of samples that are euploid for the chromosome, and $z_{max}^E$ is the maximal z-score in a set of samples that are euploid for the chromosome;
wherein, if all of (a) to (c) are fulfilled, said sample is not qualified for determining the fetal trisomy status of the chromosome, or
wherein, if any one of (a) to (c) is not fulfilled, said sample is qualified for determining the fetal trisomy status of the chromosome.

**6.** The method of claim 5, wherein, if $z > z_{max}^E$ , then the trisomy status is determined as trisomic for the chromosome.

**7.** A NIPT method for determining whether or not a sample obtained from a pregnant subject, preferably a human subject is qualified for determining the fetal monosomy status of a chromosome, comprising determining whether

(a)

$$f \leq f_{LoD}^M$$

(b)

$$t \leq (1 - 0.5 * f) * t_{max}^E$$

(c)

$$t \geq t_{min}^E$$

wherein $f$ is the fetal fraction,

t is the read representation of the chromosome,

$f_{LoD}^M$ is the limit of detection (LoD) for a monosomy of the chromosome,

$t_{min}^E$ is the minimal value of the read representation of the chromosome in a set of euploid samples, and

$t_{max}^E$ is the maximal value of the read representation of the chromosome in a set of euploid samples;

wherein, if all of (a) to (c) are fulfilled, said sample is not qualified for determining the fetal monosomy status of the chromosome, or

wherein, if any one of (a) to (c) is not fulfilled, said sample is qualified for determining the fetal monosomy status of the chromosome.

8. The method of claim 7, wherein the fetal monosomy status is monosomy 13, 18, 21, or X.

9. The method of claim 7 or 8, wherein $f_{LoD}^M$ is given by the following formula

$$f_{LoD}^M = \left(1 - \frac{t_{min}^E}{t_{max}^E}\right) * 2.$$

10. The method of any one of claims 7 to 9, wherein, if $t < t_{min}^E$, then the monosomy status is determined as monosomic for the chromosome.

11. The method of any one of claims 7 to 9, wherein the read representation of a chromosome t is replaced by the z-score z

$$z = \frac{t - \mu^E}{\sigma^E},$$

wherein $\mu^E$ is an estimate of the location of the distribution of the read representation of the chromosome in a population that is euploid in the chromosome, $\sigma^E$ is an estimate of the scale of the distribution of the read representation of the chromosome in a population that is euploid in the chromosome, comprising determining whether

(a)

$$z \leq f_{LoD,z}^M$$

(b)

$$z \geq k_f^M * z_{min}^E + \left(k_f^M - 1\right) * \mu^E/\sigma^E$$

(c)

$$z \leq z_{max}^E$$

wherein

$$f_{LoD,z}^{M} = \frac{2 * (z_{max}^{E} - z_{min}^{E})}{z_{max}^{E} + \mu^{E}/\sigma^{E}}$$

$$k_{f}^{M} = (1 - 0.5 * f)$$

$z_{min}^{E}$ is the minimal z-score in a set of samples that are euploid for the chromosome, and $z_{max}^{E}$ is the maximal z-score in a set of samples that are euploid in the chromosome;

wherein, if all of (a) to (c) are fulfilled, said sample is not qualified for determining the fetal monosomy status of the chromosome, or

wherein, if any one of (a) to (c) is not fulfilled, said sample is qualified for determining the fetal monosomy status of the chromosome.

12. The method of claim 11, wherein, if $z < z_{min}^{E}$, then the monosomy status is determined as monosomic for the chromosome.

13. The method of claim 1 and claim 7, wherein, if a sample is qualified for determining the fetal trisomy in a chromosome and the sample is qualified for determining the fetal monosomy of the chromosome and the read representation of the chromosome t is such that $t > t_{min}^{E}$ and $t < t_{max}^{E}$, then the aneuploidy status is determined as euploid for the chromosome.

14. The method of claim 5 and claim 11, wherein if a sample is qualified for determining the fetal trisomy of a chromosome and the sample is qualified for determining the fetal monosomy of the chromosome and the z-score z is such that $z > z_{min}^{E}$ and $z < z_{max}^{E}$, then the aneuploidy status is determined as euploid for the chromosome.

15. The method according to any one of the preceding claims, wherein the read representation of a chromosome t is replaced by the normalized read counts over a subset of disjoint subregions of the chromosome.

**Patentansprüche**

1. NIPT-Verfahren zur Bestimmung, ob eine Probe, die von einem schwangeren Subjekt, vorzugsweise einem humanem Subjekt, erhalten wurde, zur Bestimmung des fötalen Trisomie-Status eines Chromosoms geeignet ist oder nicht, umfassend das Bestimmen, ob

(a)

$$f \leq f_{LoD}^{T}$$

(b)

$$t \geq (1 + 0.5 * f) * t_{min}^{E}$$

(c)

$$t \leq t_{max}^{E}$$

wobei $f$ die fötale Fraktion ist,

t die gelesene Darstellung des Chromosoms ist,

$f_{LoD}^{T}$ die Nachweisgrenze (LoD) für die Trisomie des Chromosoms ist,

$t_{min}^{E}$ der minimale Wert der gelesenen Darstellung des Chromosoms in einem Satz euploider Proben ist, und

$t_{max}^E$ der maximale Wert der gelesenen Darstellung des Chromosoms in einem Satz euploider Proben ist; wobei, wenn alle von (a) bis (c) erfüllt sind, die Probe nicht zur Bestimmung des fötalen Trisomie-Status des Chromosoms geeignet ist, oder
wobei, wenn eine von (a) bis (c) nicht erfüllt ist, die Probe zur Bestimmung des fötalen Trisomie-Status des Chromosoms geeignet ist.

2. Verfahren nach Anspruch 1, wobei der fötale Trisomie-Status Trisomie 13, 18, 21 oder Triple X ist.

3. Verfahren nach Anspruch 1 oder 2, wobei $f_{LoD}^T$ durch die folgende Formel gegeben ist

$$f_{LoD}^T = \left( \frac{t_{max}^E}{t_{min}^E} - 1 \right) * 2.$$

4. Verfahren nach einem der Ansprüche 1 bis 3, wobei, wenn $t > t_{max}^E$, dann wird der fötale Trisomie-Status für das Chromosom als trisomisch bestimmt.

5. Verfahren nach einem der Ansprüche 1 bis 3, wobei die gelesene Darstellung eines Chromosoms t durch den z-Wert z

$$z = \frac{t - \mu^E}{\sigma^E}$$

ersetzt wird,
wobei $\mu^E$ eine Schätzung der Lage der Verteilung der gelesenen Darstellung des Chromosoms in einer Population ist, die im Chromosom euploid ist, $\sigma^E$ eine Schätzung der Skala der Verteilung der gelesenen Darstellung des Chromosoms in einer Population ist, die im Chromosom euploid ist, umfassend das Bestimmen, ob

$$\text{(a)} \quad z \leq f_{LoD,z}^T$$

$$\text{(b)} \quad z \geq k_f^T * z_{min}^E + \left( k_f^T - 1 \right) * \mu^E / \sigma^E$$

$$\text{(c)} \quad z \leq z_{max}^E$$

wobei

$$f_{LoD,z}^T = \frac{2 * (z_{max}^E - z_{min}^E)}{z_{min}^E + \mu^E / \sigma^E}$$

$$k_f^T = (1 + 0.5 * f)$$

$z_{min}^E$ der minimale z-Wert in einem Satz von Proben ist, die für das Chromosom euploid sind, und $z_{max}^E$ der maximale z-Wert in einem Satz von Proben ist, die für das Chromosom euploid sind;
wobei, wenn alle von (a) bis (c) erfüllt sind, die Probe nicht zur Bestimmung des fötalen Trisomie-Status des Chromosoms geeignet ist, oder
wobei, wenn eine von (a) bis (c) nicht erfüllt ist, die Probe zur Bestimmung des fötalen Trisomie-Status des Chromosoms geeignet ist.

6. Verfahren nach Anspruch 5, wobei, wenn $z > z_{max}^E$, dann wird der Trisomie-Status für das Chromosom als trisomisch bestimmt.

7.  NIPT-Verfahren zur Bestimmung, ob eine Probe, die von einem schwangeren Subjekt, vorzugsweise einem humanem Subjekt, erhalten wurde, zur Bestimmung des fötalen Monosomie-Status eines Chromosoms geeignet ist oder nicht, umfassend das Bestimmen, ob

(a)

$$f \leq f_{LoD}^{M}$$

(b)

$$t \leq (1 - 0.5 * f) * t_{max}^{E}$$

(c)

$$t \geq t_{min}^{E}$$

wobei $f$ die fötale Fraktion ist,
t die gelesene Darstellung des Chromosoms ist,
$f_{LoD}^{M}$ die Nachweisgrenze (LoD) für die Monosomie des Chromosoms ist,
$t_{min}^{E}$ der minimale Wert der gelesenen Darstellung des Chromosoms in einem Satz euploider Proben ist, und
$t_{max}^{E}$ der maximale Wert der gelesenen Darstellung des Chromosoms in einem Satz euploider Proben ist;
wobei, wenn alle von (a) bis (c) erfüllt sind, die Probe nicht zur Bestimmung des fötalen Monosomie-Status des Chromosoms geeignet ist, oder
wobei, wenn eine von (a) bis (c) nicht erfüllt ist, die Probe zur Bestimmung des fötalen Monosomie-Status des Chromosoms geeignet ist.

8.  Verfahren nach Anspruch 7, wobei der fötale Trisomie-Status Monosomie 13, 18, 21 oder X ist.

9.  Verfahren nach Anspruch 7 oder 8, wobei $f_{LoD}^{M}$ durch die folgende Formel gegeben ist

$$f_{LoD}^{M} = \left(1 - \frac{t_{min}^{E}}{t_{max}^{E}}\right) * 2.$$

10.  Verfahren nach einem der Ansprüche 7 bis 9, wobei, wenn $t < t_{min}^{E}$, dann wird der Monosomie-Status für das Chromosom als monosomisch bestimmt.

11.  Verfahren nach einem der Ansprüche 7 bis 9, wobei die gelesene Darstellung eines Chromosoms t durch den z-Wert z

$$z = \frac{t - \mu^{E}}{\sigma^{E}}$$

ersetzt wird,
wobei $\mu^{E}$ eine Schätzung der Lage der Verteilung der gelesenen Darstellung des Chromosoms in einer Population ist, die im Chromosom euploid ist, $\sigma^{E}$ eine Schätzung der Skala der Verteilung der gelesenen Darstellung des Chromosoms in einer Population ist, die im Chromosom euploid ist, umfassend das Bestimmen, ob

(a)

$$z \leq f_{LoD,z}^{M}$$

(b)

$$z \geq k_f^M * z_{min}^E + \left(k_f^M - 1\right) * \mu^E / \sigma^E$$

(c)

$$z \leq z_{max}^E$$

wobei

$$f_{LoD,z}^M = \frac{2 * (z_{max}^E - z_{min}^E)}{z_{max}^E + \mu^E / \sigma^E}$$

$$k_f^M = (1 - 0.5 * f)$$

$z_{min}^E$ der minimale z-Wert in einem Satz von Proben ist, die für das Chromosom euploid sind, und $z_{max}^E$ der maximale z-Wert in einem Satz von Proben ist, die in dem Chromosom euploid sind;

wobei, wenn alle von (a) bis (c) erfüllt sind, die Probe nicht zur Bestimmung des fötalen Monosomie-Status des Chromosoms geeignet ist, oder

wobei, wenn eine von (a) bis (c) nicht erfüllt ist, die Probe zur Bestimmung des fötalen Monosomie-Status des Chromosoms geeignet ist.

12. Verfahren nach Anspruch 11, wobei, wenn $z < z_{min}^E$ , dann wird der Monosomie-Status für das Chromosom als monosomisch bestimmt.

13. Verfahren nach Anspruch 1 und Anspruch 7, wobei, wenn eine Probe zur Bestimmung der fötalen Trisomie in einem Chromosom geeignet ist und die Probe zur Bestimmung der fötalen Monosomie des Chromosoms geeignet ist und die gelesene Darstellung des Chromosoms *t* derart ist, dass $t > t_{min}^E$ und $t < t_{max}^E$ , dann wird der Aneuploidie-Status für das Chromosom als euploid bestimmt.

14. Verfahren nach Anspruch 5 und Anspruch 11, wobei, wenn eine Probe zur Bestimmung der fötalen Trisomie eines Chromosoms geeignet ist und die Probe zur Bestimmung der fötalen Monosomie des Chromosoms geeignet ist und der z-Wert z derart ist, dass $z > z_{min}^E$ und $z < z_{max}^E$ , dann wird der Aneuploidie-Status für das Chromosom als euploid bestimmt.

15. Verfahren nach einem der vorstehenden Ansprüche, wobei die gelesene Darstellung eines Chromosoms t durch die normalisierte Lesezählung über eine Teilmenge disjunkter Teilbereiche des Chromosoms ersetzt wird.

**Revendications**

1. Procédé NIPT pour déterminer si un échantillon obtenu à partir d'un sujet enceinte, de préférence un sujet humain, est qualifié ou non pour déterminer le statut de trisomie foetale d'un chromosome, comprenant le fait de déterminer si

(a)

$$f \leq f_{LoD}^T$$

(b)

$$t \geq (1 + 0.5 * f) * t_{min}^E$$

(c)

$$t \leq t_{max}^E$$

où $f$ est la fraction fœtale,

t est la représentation lue du chromosome,

$f_{LoD}^T$ est la limite de détection (LD) pour la trisomie du chromosome,

$t_{min}^E$ est la valeur minimale de la représentation lue du chromosome dans un ensemble d'échantillons euploïdes, et

$t_{max}^E$ est la valeur maximale de la représentation lue du chromosome dans un ensemble d'échantillons euploïdes;

dans lequel, si toutes les conditions (a) à (c) sont remplies, ledit échantillon n'est pas qualifié pour déterminer le statut de trisomie foetale du chromosome, ou

dans lequel, si l'une quelconque des conditions (a) à (c) n'est pas remplie, ledit échantillon est qualifié pour déterminer le statut de trisomie foetale du chromosome.

2. Procédé de la revendication 1, dans lequel le statut de trisomie du foetus est la trisomie 13, 18, 21 ou triple X.

3. Procédé selon les revendications 1 ou 2, dans lequel $f_{LoD}^T$ est donné par la formule suivante:

$$f_{LoD}^T = \left( \frac{t_{max}^E}{t_{min}^E} - 1 \right) * 2.$$

4. Procédé de l'une quelconque des revendications 1 à 3, dans lequel, si $t > t_{max}^E$, alors le statut de trisomie du foetus est déterminé comme étant trisomique pour le chromosome.

5. Procédé de l'une quelconque des revendications 1 à 3, dans lequel la représentation lue d'un chromosome t est remplacée par le z-score z

$$z = \frac{t - \mu^E}{\sigma^E},$$

dans laquelle $\mu^E$ est une estimation de l'emplacement de la distribution de la représentation lue du chromosome dans une population qui est euploïde dans le chromosome, $\sigma^E$ est une estimation de l'échelle de la distribution de la représentation lue du chromosome dans une population qui est euploïde dans le chromosome, comprenant la détermination du fait que

(a)

$$z \leq f_{LoD,z}^T$$

(b)

$$z \geq k_f^T * z_{min}^E + \left( k_f^T - 1 \right) * \mu^E / \sigma^E$$

(c)

$$z \leq z_{max}^E$$

dans lequel

$$f_{LoD,z}^T = \frac{2 * (z_{max}^E - z_{min}^E)}{z_{min}^E + \mu^E/\sigma^E}$$

$$k_f^T = (1 + 0.5 * f)$$

$z_{min}^E$ est le score z minimal dans un ensemble d'échantillons qui sont euploïdes pour le chromosome, et $z_{max}^E$ est le score z maximal dans un ensemble d'échantillons qui sont euploïdes pour le chromosome ;

dans lequel, si toutes les conditions (a) à (c) sont remplies, ledit échantillon n'est pas qualifié pour déterminer le statut de trisomie foetale du chromosome, ou

dans lequel, si l'une quelconque des conditions (a) à (c) n'est pas remplie, ledit échantillon est qualifié pour déterminer le statut de trisomie foetale du chromo-some.

6. Procédé de la revendication 5, dans lequel, si $z > z_{max}^E$ , alors le statut de trisomie est déterminé comme étant trisomique pour le chromosome.

7. Procédé NIPT pour déterminer si un échantillon obtenu à partir d'un sujet enceinte, de préférence un sujet humain, est qualifié ou non pour déterminer le statut de monosomie foetale d'un chromosome, comprenant la détermination de ce qui suit

(a)

$$f \leq f_{LoD}^M$$

(b)

$$t \leq (1 - 0.5 * f) * t_{max}^E$$

(c)

$$t \geq t_{min}^E$$

où $f$ est la fraction fœtale,

t est la représentation lue du chromosome,

$f_{LoD}^M$ est la limite de détection (LD) pour une monosomie du chromosome,

$t_{min}^E$ est la valeur minimale de la représentation lue du chromosome dans un ensemble d'échantillons euploïdes, et

$t_{max}^E$ est la valeur maximale de la représentation lue du chromosome dans un ensemble d'échantillons euploïdes ;

dans lequel, si toutes les conditions (a) à (c) sont remplies, ledit échantillon n'est pas qualifié pour déterminer le statut de monosomie foetale du chromo-some, ou

dans lequel, si l'une quelconque des conditions (a) à (c) n'est pas remplie, ledit échantillon est qualifié pour déterminer le statut de monosomie foetale du chromosome.

8. Procédé de la revendication 7, dans lequel le statut de monosomie fœtale est la monosomie 13, 18, 21 ou X.

9. Procédé selon la revendication 7 ou 8, dans lequel $f_{LoD}^M$ est donné par la formule suivante

$$f_{LoD}^{M} = \left(1 - \frac{t_{min}^{E}}{t_{max}^{E}}\right) * 2.$$

**10.** Procédé de l'une quelconque des revendications 7 à 9, dans lequel, si t < $t^{\wedge}in,$ alors le statut de monosomie est déterminé comme étant monosomique pour le chromosome.

**11.** Procédé de l'une quelconque des revendications 7 à 9, dans lequel la représentation lue d'un chromosome $t$ est remplacée par le z-score z

$$z = \frac{t - \mu^{E}}{\sigma^{E}},$$

dans laquelle $\mu^{E}$ est une estimation de l'emplacement de la distribution de la représentation lue du chromosome dans une population qui est euploïde dans le chromosome, $\sigma^{E}$ est une estimation de l'échelle de la distribution de la représentation lue du chromosome dans une population qui est euploïde dans le chromosome, comprenant la détermination du fait que

(a)

$$z \leq f_{LoD,z}^{M}$$

(b)

$$z \geq k_{f}^{M} * z_{min}^{E} + \left(k_{f}^{M} - 1\right) * \mu^{E}/\sigma^{E}$$

(c)

$$z \leq z_{max}^{E}$$

où

$$f_{LoD,z}^{M} = \frac{2 * (z_{max}^{E} - z_{min}^{E})}{z_{max}^{E} + \mu^{E}/\sigma^{E}}$$

$$k_{f}^{M} = (1 - 0.5 * f)$$

$z_{min}^{E}$ est le score z minimal dans un ensemble d'échantillons qui sont euploïdes pour le chromosome, et

$z_{max}^{E}$ est le score z maximal dans un ensemble d'échantillons qui sont euploïdes pour le chromosome ;
dans lequel, si toutes les conditions (a) à (c) sont remplies, ledit échantillon n'est pas qualifié pour déterminer le statut de monosomie foetale du chromosome, ou
dans lequel, si l'une quelconque des conditions (a) à (c) n'est pas remplie, ledit échantillon est qualifié pour déterminer le statut de monosomie foetale du chromosome.

**12.** Procédé selon la revendication 11, dans lequel, si $z < z_{min}^{E}$ » comme étant monosomique pour le chromosome.

**13.** Procédé selon la revendication 1 et selon la revendication 7, dans lequel, si un échantillon est qualifié pour déterminer la trisomie foetale dans un chromosome et l'échantillon est qualifié pour déterminer la monosomie foetale du chromosome et la représentation lue du chromosome t est telle que et $t > t_{min}^{E}$ $t < t_{max}^{E}$ , alors l'état d'aneuploïdie est déterminé comme étant euploïde pour le chromosome.

**14.** Procédé selon la revendication 5 et selon la revendication 11, dans lequel si un échantillon est qualifié pour déterminer la trisomie foetale d'un chromosome et l'échantillon est qualifié pour déterminer la monosomie foetale du chromosome et le score z est tel que $z > z_{min}^E$ et $z > z_{max}^E$ alors l'état d'aneuploïdie est déterminé comme étant euploïde pour le chromosome.

**15.** Procédé selon l'une quelconque des revendications précédentes, dans lequel la représentation des lectures d'un chromosome t est remplacée par les comptes de lecture normalisés sur un sous-ensemble de sous-régions disjointes du chromosome.

**Fig. 1**

**Fig. 2**

**Fig. 3**

**Fig. 4**

# Fig. 5

(c) Polygon of indeterminacy

(b) $\triangle^M$

(a) $\triangle^T$

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2016224724 A
- EP 2981921 A
- EP 994963 A
- EP 1981995 A
- EP 2183693 A
- EP 2514842 A
- WO 2017050244 A
- WO 9844151 A
- WO 06084132 A

### Non-patent literature cited in the description

- **LO et al.** *The Lancet,* 1997, vol. 350, 485-487
- **BAYINDIR et al.** *Eur. J. Hum. Genet.,* 2015, vol. 23, 1286-1293
- **PORRECO et al.** *Am. J. Obstet. Gynecol.,* 2014, vol. 211, 365-e1
- **THUNG et al.** *Expert. Rev. Mol. Diagn.,* 2015, vol. 15, 111-124
- **TAMMINGA et al.** *Adv. Clin. Chem.,* 2016, vol. 74, 63-102
- **CHIU et al.** *Proc. Natl. Acad. Sci.,* 2008, vol. 105, 20458-20463
- **JENSEN et al.** *PloS One,* 2013, vol. 8, e57381
- **CHANDRANANDA et al.** *PloS One,* 2014, vol. 9, e86993
- **SEHNERT et al.** *Clin. Chem.,* 2011, vol. 57, 1042-1049
- **ZHAO et al.** *Clin. Chem.,* 2015, vol. 61, 608-616
- **YEANG et al.** *Ultrasound Obstet. & Gynecol.,* 2014, vol. 44, 25-30
- **JOHANSSON et al.** *Sci. Reports,* 2017, vol. 7
- **SIKKEMA-RADDATZ et al.** *Sci. Reports,* 2016, vol. 6, 38359
- **BALSLEV-HARDER et al.** *Prenat. Diagn.*
- **PALOMAKI et al.** *Genet. Medicine,* 2011, vol. 13, 913-920
- **CANICK et al.** *Prenat. Diagn.,* 2013, vol. 33, 667-674
- **EHRICH et al.** *Am. J. Obstet. Gynecol.,* 2011, vol. 204, 205-e1
- **NORTON et al.** *Am. J. Obstet. Gynecol.,* 2012, vol. 207, 137-e1
- **ASHOOR et al.** *Ultrasound Obstet. & Gynecol.,* 2013, vol. 41, 26-32
- **FIORENTINO et al.** *Prenat. Diagn.,* 2016, vol. 36, 304-311, http://dx.doi.org/10.1002/pd.4780
- **VAN BEEK et al.** *PRENATAL DIAGNOSIS,* 2017, vol. 8, 769-773
- **MACKIE et al.** *BJOG: An Int. J. Obstet. & Gynaecol.,* 2017, vol. 124, 32-46
- **GIL et al.** *Fetal Diagn. Ther.,* 2014, vol. 35, 156-173
- **TAYLOR-PHILLIPS et al.** *BMJ Open,* 2016, vol. 6, e010002
- **TAMMINGA et al.** *Advances in clinical chemistry,* 2016, vol. 74, 63-102
- **NORWITZ et al.** *Rev Obstet Gynecol,* 2013, vol. 6 (2), 48-62
- **BARRETT et al.** *PloS One,* 2011, vol. 6, e25202
- **KIM et al.** *Prenat. Diagn.,* 2015, vol. 35, 810-815
- **LO.** *Open Biol.,* 2012, vol. 2 (6
- **NIGAM et al.** *JIMSA,* 2012, vol. 25, 119-200
- **CHIU.** *BMJ,* 2011, vol. 342 (c7401
- **HUDECOVA et al.** *PLoS One,* 2014, vol. 9, e88484
- **LIAO et al.** *Clin Chem,* 2011, vol. 57, 91-101
- **LO et al.** *Sci Transl Med,* 2010, vol. 2, 61ra91
- **YU et al.** *PNAS,* 2014, vol. 111, 8583-8588
- **LANGMEAD et al.** *Nat Methods,* 2012, vol. 9, 357-359
- **DUNCAN et al.** *Diagn Mol Pathol,* 2017
- **VOELKERDING et al.** *Clin Chem,* 2009, vol. 55 (4), 641-658
- **WHEELER et al.** *Nature,* 2008, vol. 452 (7189), 872-876
- **CANARD et al.** *Gene,* 1994, vol. 148 (1), 1-6
- **FENG et al.** *Mol Ecol Res,* 2015, vol. 16 (1), 91-102
- **SCHUSTER.** *Nat Methods,* 2008, vol. 51 (1), 16-18
- **DRMANAC et al.** *Science,* 2010, vol. 327 (5961), 78-81
- **PORRECA.** *Nat Biotechnol,* 2010, vol. 28 (1), 43-44
- **HARRIS et al.** *Science,* 2008, vol. 320 (5872), 106-109

- **LEVENE et al.** *Science,* 2003, vol. 299 (5607), 982-686
- **EID et al.** *Science,* 2009, vol. 323 (5910), 133-138
- **NIEDRINGHAUS et al.** *Anal Chem,* 2011, vol. 83 (12), 4327-4341
- **GREININGER et al.** *Genome Medicine,* 2015, vol. 7, 99
- **MCCULLUGH et al.** *PloS One,* 2014
- **LANGMEAD et al.** *Nat. Methods,* 2012, vol. 9, 357-359
- **HUBER et al.** *Nat. Methods,* 2015, vol. 12, 115-121, http://dx.doi.org/10.1038/nmeth.3252
- **MORGAN et al.** Rsamtools: Binary alignment (BAM), FASTA, variant call (BCF), and tabix file import. *R package version 1.18.3, http://bioconductor.org/packages/release/bioc/html/Rsamtools.html*
- **HUDECOVA et al.** *PloS One,* 2014, vol. 9, e88484
- **CHIU et al.** *BMJ,* 2011, vol. 342, c7401
- **GRENDAR et al.** *Prenat. Diagn.,* 2017
- **VAN BEEK et al.** *Prenat. Diagn.,* 2017, vol. 37, 769-773
- **PENG et al.** *Int. J. Mol. Sci.,* 2017, vol. 18, 453
- **R CORE TEAM.** R: A Language and Environment for Statistical Computing. R Foundation for Statistical Computing, 2015